(19) Europäisches Patentamt
European Patent Office
Office européen des brevets

(11) Numéro de publication: **0 104 984**
**B1**

(12) # FASCICULE DE BREVET EUROPEEN

(45) Date de publication du fascicule du brevet: **16.05.90**

(21) Numéro de dépôt: **83401766.7**

(22) Date de dépôt: **09.09.83**

(51) Int. Cl.[5]: **C 07 C 269/06,**
C 07 D 295/20,
C 07 D 249/18,
C 07 D 211/78,
C 07 D 273/00,
C 07 D 489/08, C 07 D 451/10

(54) **Procédé de préparation des carbamates vinyliques et nouveaux carbamates vinyliques.**

(30) Priorité: **24.09.82 US 423465**

(43) Date de publication de la demande:
**04.04.84 Bulletin 84/14**

(45) Mention de la délivrance du brevet:
**16.05.90 Bulletin 90/20**

(84) Etats contractants désignés:
**AT BE CH DE FR GB IT LI LU NL SE**

(56) Documents cités:
**THE JOURNAL OF ORGANIC CHEMISTRY, vol. 27, no. 12, décembre 1962, pages 4331-4337, American Chemical Society, Washington, DC, US; C.G. OVERBERGER et al.: " Preparation and polymerisation of S-,O-, and N-vinyl derivatives of carbonic acid. Unsaturated carbonic acid derivatives"**

**TETRAHEDRON LETTERS, vol. 22, no. 26, 1981, pages 2431-2434, Pergamon Press Ltd., Oxford, GB; R.F. PRATT et al.: "Ring opening and closing reactions of imidazoles and other 1,3-diazaheterocycles with vinyl chloroformate and phenyl chloroformate"**

(73) Titulaire: **SOCIETE NATIONALE DES POUDRES ET EXPLOSIFS**
**12, quai Henri IV**
**F-75181 Paris Cédex 04 (FR)**

(72) Inventeur: **Olofson, Roy Arne**
**529 Cricklewood Drive**
**State College Pennsylvania 16801 (US)**
Inventeur: **Wooden, Gary Paul**
**508 South Fraser Street**
**State College Pennsylvania 16801 (US)**
Inventeur: **Martz, Jonathan Thomas**
**1441 Wightman Street**
**Pittsburgh Pennsylvania 15217 (US)**

**EP 0 104 984 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention se rapporte à une procédé de préparation des carbamates vinyliques. Elle concerne aussi de nouveaux carbamates vinyliques qui sont utiles comme pesticides agricoles, herbicides, insecticides et produits apparentés, ainsi que comme intermédiaires dans la préparation de produits pharmaceutiques, ou encore, qui conduisent, après polymérisation, à des matériaux intéressants.

Au cours des vingt-cinq dernières années, divers procédés de préparation des carbamates vinyliques ont été décrits dans la littérature spécialisée. Selon la brevet françois n° 1 478 633 de la DOW CHEMICAL COMPANY, certains carbamates vinyliques N-hétérocycliques sont préparés par réaction d'un haloformiate vinylique et d'une amine secondaire N-hétérocyclique. Mais les haloformiates de vinyle qui constituent des matières de départ sont des substances très difficiles à préparer. Ainsi, le procédé décrit dans le brevet américain n° 2 377 085 qui consiste à pyrolyser du bischloroformiate de glycol à 450°C, ne permet pas d'obtenir plus de 11% de chloroformate de vinyle. SCHAEFGEN (dans le brevet américain 3 118 862) et LEE (dans le Journal of Organic Chemistry, vol. 30, p.3943 — 1965) ont amélioré cette synthèse; toutefois, ces améliorations n'aboutissent qu'à des rendements respectivement de 30 et 44%. L'une des difficultés recontrées est le bouchage des réacteurs tubulaires. Une autre difficulté consiste dans le fait que les sous-produits sont toxiques et cancérigènes. Un nouveau procédé a été développé dans les laboratoires de la SOCIETE NATIONALE DES POUDRES ET EXPLOSIFS. Il consiste à faire réagir du phosgène avec un sel de mercure. Ce procédé est décrit dans la brevet américain 4 210 598. Il permet d'obtenir un meilleur rendement en chloroformiates de vinyle, et d'utiliser ceux-ci sur une grande échelle pour la préparation des carbamates. Cependant, même ce procédé-là présente certain inconvénients, notamment du fait de l'utilisation des sels de mercure: ceux-ci sont coûteux et requièrent des précautions particulières.

Un autre procédé de préparation des carbamates vinyliques consiste à déshydrohalogéner des carbamates de β-chloroéthyle. Ce procédé est décrit dans le Journal of Organic Chemistry, vol. 27, p. 4331 (1962). Les rendements sont faibles et de nombreux sous-produits sont obtenus. La déshydrohalogénation n'a lieu qu'en présence de butylate tertiaire de potassium qui est un réactif cher et difficile à manipuler. Aussi ce procédé est-il difficilement applicable à l'échelle industrielle.

Dans le Journal of Chemistry and Industry (numéro du 8 Février 1969, p. 166), FRANKO-FILIPASIC et PATARCITY rapportent qu'ils ont obtenu, par réaction d'un petit nombre de cétones et d'un aldéhyde avec des chlorures de dialkylcarbamoyle, quelques carbamates vinyliques avec des rendements également faibles. Ces chercheurs formulent l'hypothèse qu'un produit intermédiaire, tel qu'un carbamate d'α-chloroalkyle, est formé et rapidement déshydrohalogéné; cependant, ils n'ont pas détecté la moindre trace de cet intermédiaire, et n'ont pas réussi à accroître la vitesse de la réaction en ajoutant divers catalyseurs. OLOFSON et CUOMO, dan Tetrahedron Lett., vol. 21, 819 (1980) rapportent de leur côté la synthèse de la N-(E,Z-propényloxycarbonyl)morpholine, réalisée au moyen d'un fluorocarbamate en le faisant réagir avec un éther de triméthylsilyle, qui est un réactif coûteux.

Récemment, STANG et ANDERSON, dans le Journal of Organic Chemistry, vol. 46, p.4585 (1981) rapportent qu'ils ont à nouveau essayé de préparer des carbamates vinyliques, mais par une voie nouvelle, cette fois-ci consistant à utiliser des isocyanates et en passant par l'intermédiaire de carbènes. Cependant, cette synthèse présente elle aussi des inconvénients car elle requiert la présence d'un fluorure, qui est un réactif de laboratoire très coûteux et inapproprié pour les opérations à grande échelle. Par ailleurs, seulement quelques milligrammes du produit recherché ont été obtenus.

Ce bref aperçu montre que, au moins pendant ces vingt-cinq dernières années, le besoin d'un procédé simpe et économique de préparation des carbamates de vinyle, utilisable à l'échelle industrielle, s'est manifesté. De même qu'un besoin d'obtention de nouveaux carbamates vinyliques.

L'un des objectifs de la présente invention est la préparation des carbamates vinyliques par un procédé simple et économique, applicable à l'échelle industrielle et fournissant les produits recherchés avec de bons rendements.

Un autre objectif de cette invention est de préparer de nouveaux carbamates vinyliques, utiles comme parfums, pesticides et monomères et également des carbamates connus qui, après polymérisation, présentent un intérêt en tant que substances moulables suivant la description de brevet français 1 478 633 et du Journal of Organic Chemistry, vol. 27, p. 4331 (1962).

Plus précisément, la présente invention concerne un procédé de préparation des carbamates vinyliques de formula I:

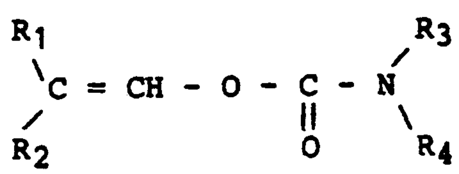

(I)

dans laquelle $R_1$ et $R_2$, identiques ou différents représentent

un atome d'hydrogène,

un radical aliphatique saturé ou non, substitué ou non, ou bien

$R_1$ et $R_2$, conjointement avec l'atome de carbone auquel ils sont attachés, forment un cycle qui est saturé ou non, substitué ou non,

$R_3$ et $R_4$ sont identiques ou différents et représentent

un atome d'hydrogène,

un radical aliphatique, cycloaliphatique, substitué ou non, saturé ou non, l'insaturation se trouvant sur un atomes de carbone qui n'est pas adjacent à l'atome d'azote,

un radical de formule

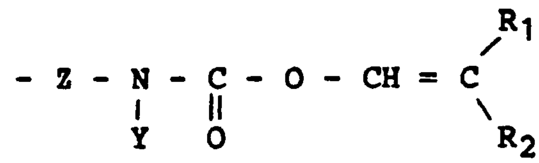

dans laquelle Y est un radical alkyle, $R_1$ et $R_2$ sont définis comme ci-dessus et Z est un chaîne hydrocarbonée comportant 2 à 20 atomes de carbone ou Z est un chaîne hydrocarbonée constituée de 2 à 20 atomes de carbone et contenant en outre des hétéroatomes W, W étant O,S,NR'', chaque hétéroatome étant séparé d'un autre hétéroatome par au moins deux atomes de carbone et R'' étant un radical hydrocarboné ou Z est un groupe

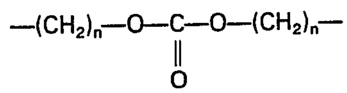

et n est un nombre entier compris entre 2 et 10,

un radical aromatique substitué ou non ou bien,

$R_3$ et $R_4$ forment conjointement avec l'atome d'zote auquel ils sont attachés, un hétérocycle saturé ou non, substitué ou non,

$R_3$ et $R_4$ forment conjointement avec l'atome d'azote auquel ils sont attachés, un hétérocycle qui fait partie d'un système cyclique condensé saturé ou non, substitué ou non,

$R_3$ et $R_4$ forment conjointement avec l'atome d'azote auquel ils sont attachés, un hétérocycle qui contient au moins un autre hétéroatome qui est O, S, N ou $NR_x$, $R_x$ étant un radical hydrocarboné, et/ou un atome d'azote qui forme un groupe carbamate vinylique,

$R_3$ et $R_4$ forment conjointement avec l'atome d'azote auquel ils sont attachés, un cycle de formule

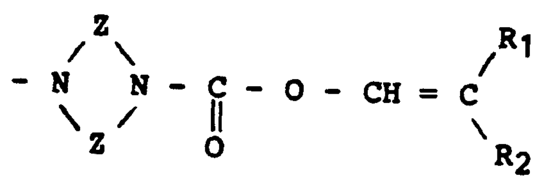

dans laquelle Z, $R_1$ et $R_2$ ont la signification précédente,

$R_3$ et $R_4$ forment conjointement avec l'atome d'azote auquel ils sont attachés un cycle pipérazinique les deux atomes d'azote du cycle pipérazinique portant le groupe de formule:

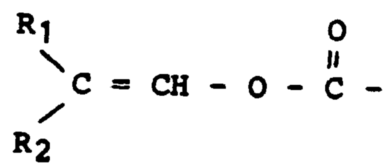

dans laquelle $R_1$ et $R_2$ ont la signification précédente,

Il faut noter que $R_3$ et $R_4$ peuvent contenir des groupes cétoniques, esters d'alcools primaires, secondaires et tertiaires et de méthanol, esters conjugués, carbonates, éthers y compris éthers aromatiques et des groupes fonctionnels amides. Il peuvent aussi contenir des sels d'ammonium quaternaire.

Selon une réalisation particulière de la présente invention $R_1$ et $R_2$ sont identiques ou différents, et représentent:

un atome d'hydrogène,

un radical aliphatique, saturé ou non saturé, substitué ou non substitué,

ou bien $R_1$ et $R_2$, conjointement avec l'atome de carbone auquel ils sont attachés, forment un cycle qui est saturé ou non saturé, substitué ou non substitué.

$R_3$ et $R_4$ sont identiques ou différents et représentent:

un atome d'hydrogène,

un radical aliphatique, cycloaliphatique ou hétérocyclique, substitué ou non substitué, saturé ou non saturé, l'insaturation ne se trouvant pas sur un atome de carbone adjacent à l'atome d'azote,

un radical de formule

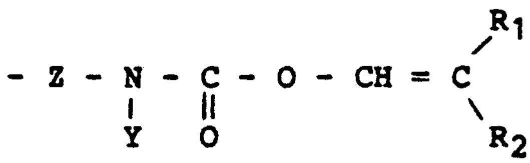

dans laquelle:

Z est un chaîne comportant 2 à 6 atomes de carbone,

Y est un radical alkyle, et

$R_1$ et $R_2$ sont définis comme ci-dessus,

un radical aromatique substitué ou non ou bien

$R_3$ et $R_4$ forment conjointement avec l'atome d'zote auquel ils sont attachés, un hétérocycle,

$R_3$ et $R_4$ forment conjointement avec l'atome d'azote auquel ils sont attachés, un hétérocycle qui fait partie d'un système cyclique condensé,

$R_3$ et $R_4$ forment conjointement avec l'atome d'azote auquel ils sont attachés, un hétérocycle qui contient au moins un autre hétéro-atome,

$R_3$ et $R_4$ forment conjointement avec l'atome d'azote auquel ils sont attachés, un hétérocycle qui contient au moins un atome d'oxygène et au moins un autre atome d'azote,

$R_3$ et $R_4$ forment conjointement avec l'atome d'azote auquel ils sont attachés, un cycle pipérazinique, les deux atomes d'azote du cycle pipérazinique étant porteurs d'un groupe de formule:

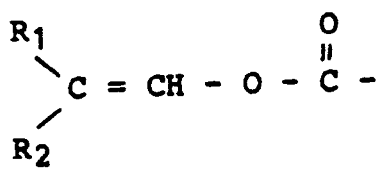

Le procédé de préparation des carbamates vinyliques de formule I ci-dessus conformément à la présente invention, comprend le chauffage d'un carbamate α-halogéné de formule II:

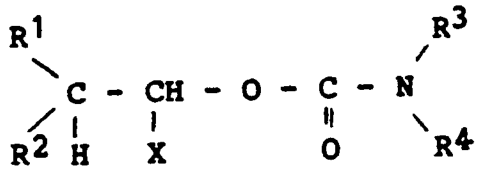

(II)

dans laquelle $R_1$, $R_2$, $R_3$ et $R_4$ sont définis comme précédemment et quand dans le composé de formule (I), $R_3$ et $R_4$ repréentant un radical qui contient un groupe carbamate vinylique, ce dernier est présent dans le composé de formula (II) sous la forme d'un carbamate saturé contenant un atome d'hydrogène en position bêta et X en position alpha, X est un atome d'halogène, à une température comprise entre 80°C et 200°C pendant une période comprise entre quelques minutes et plusieurs heures.

Le procédé selon la présente peut aussi être représenté par l'équation ci-dessous:

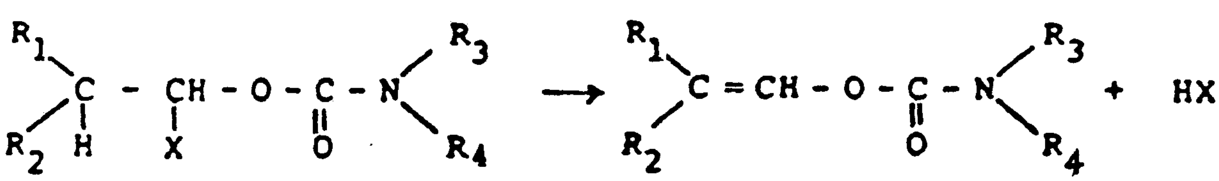

Comme l'indique cette équation, le procédé selon l'invention permet d'utiliser des composés de départ qui sont faciles à préparer, ainsi que des installations qui ne sont ni très sophistiquées, ni très coûteuses. Il permet d'obtenir les carbamates vinyliques avec un bon rendement et sans produits secondaires, difficiles à séparer ou à éliminer.

Un avantage important de la présente invention est qu'elle permet de disposer de nouveaux composés qui étaient inaccessibles jusqu'à maintenant et qui présentent un intérét en tant que pesticides agricoles, herbicides, insecticides et produits apparentés. Un autre avantage est que ces composés contiennent une double liaison carbone — carbone, qui peut être utilisée pour des réactions d'addition avec un grand nombre de composés différents. Enfin, compte tenu de la présence de la double liaison, its peuvent être polymérisés pour préparer de nouveaux composés, tels que des matières plastiques organiques ayant des propriétés similaires à celles du verre.

L'invention est décrite ci-dessous plus en détail. En se référant à la formule II, qui représente le carbamate servant de composé de départ, X est de préférence du chlore ou du brome de sorte que les composés soumis à la déshydrohalogenation sont de préférence les carbamates α-chlorés ou α-bromés. Pour le préparation des composés α-halogénés, il est possible d'utiliser le procédé décrit dans le brevet américain n° 280,241 déposé le 6 Juillet 1981 au nom de T. MALFROOT, M. PITEAU et J. P. SENET, qui permet d'utiliser sans problèmes des amines tertiaires disponibles ainsi que des chloroformiates α-halogénés, comme composés de départ. Cest composés peuvent également être préparés en faisant réagir ces mêmes chloroformiates α-halogénés avec l'ammoniac, des amines primaires ou des amines secondaires en présence d'un agent agissant comme accepteur de l'acide halohydrique formé. Les chloroformiates α-chlorés peuvent être préparés par chloration photochimique des chloroformiates correspondants ou mieux encore, par réaction du phosgène avec un aldéhyde en présence d'un catalyseur tel qu'il est décrit dans le brevet européen n° 40 153.

Une caractéristique essentielle du procédé selon la présente invention réside dans le fait que les carbamates α-halogénés de formule II sont chauffés à une température comprise entre 80°C et 200°C.

Selon l'une des variantes de l'invention, on ajoute au milieu réactionnel au moins un catalyseur, qui est un sel capable de s'ioniser facilement et dont l'anion n'est pas ou est seulement faiblement nucléophile. Le cation peut être un cation métallique, et de préférence un cation métallique alcalin ou alcalinoterreux. Il est avantageux d'associer le cation métallique de manière à former un complexe, tel qu'un éther-couronne ou un cryptant. Le cation peut également être un cation organique. Ce sera de préférence l'un des oniums, tels que l'ammonium, le phosphonium, l'arsonium ou le sulfonium, et en particulier, ce sera un cation onium substitué par au moins au radical et particulierèment par plus d'un radical ayant au moins quatre atomes de carbone. Les cations que l'on préfère sont les ions d'ammonium quaternaire.

L'anion est un halogénure, de préférence un chlorure ou bromure, ou un autre anion nucléophile ou faiblement nucléophile, tel que par exemple le $ClO_4^-$ ou le $NO_3^-$.

Il est possible d'utiliser comme catalyseur le chlorure de lithium, sodium, potassium, magnésium et calcium, ainsi que la bromure de lithium et le fluorure de potassium. Le chlorure de potassium associé au 18-couronne-6 ou 2,2,2-cryptant donne de très bons résultats. Les halogénures d'ammonium quaternaire font partie ds catalyseurs que l'on préfère. On peut citre ici le chlorure ou bromure de benzyltributylammonium, le chlorure ou bromure de tétrahexylammonium, et en particulier le bromure de tétra-n-butylammonium. Le catalyseur peut être ajouté en quantité comprise entre 0,02 et 0,5 équivalents, et de préférence entre 0,05 et 0,15 équivalents, par rapport à chaque groupe fonctionnel carbamate qui doit réagir.

Selon une seconde variante de l'invention, la réaction est effectuée en présence d'un agent capable de neutraliser l'acide halohydrique produit au cours de la réaction. Sont particulièrement appropriées à cette fin, dans le cadre de la présente invention, les substances qui sont des accepteurs d'acides et qui ne présentent pas une activité nucléophile importante, mais qui constituent des bases suffisamment fortes pour donner lieu à complex avec l'acide formé. Les substances préférées sont choisies dans le groupe qui comprend des pyridines substituées dans la position 2,4 ou 2,4,6 par des radicaux aliphatiques qui peuvent comporter un atome de carbone, par exemple, un groupe méthyle, et jusqu'à n atomes de carbone, n étant un nombre suffisamment élevé pour que le radical puisse être une chaîne polymère; les anilines substituées sur l'atome d'azote par des alkyles comportant entre un et n atomes de carbone, n étant un nombre suffisamment élevé pour le radical puisse être une chaîne polymère et en particulier les anilines substituées dont le noyau aromatique est désactivé par des substituants électrophiles, par exemple par des atomes d'halogènes, plus particulièrement en position para; certains alcènes, tels que le pinène ou le cyclododécatriène, les diisocyanates aromatiques tels que diisocyanate de toluène ou encore les diisocyanates aliphatiques tels que ceux de formule:

$$O{=}C{=}N(CH_2)_xN{=}C{=}O$$

dans laquelle x est nombre entier compris entre 6 et 36; ainsi que les carbonates alcalins ou alcalinoterreux.

Sont particulièrement appropriés la collidine, les N,N-dialkylanilines-p-halogénées et le pinène.

L'accepteur d'acide est utilisé en quantité égale ou supérieure à la quantité stoechiométrique, et de préférence en excédent léger ou notable.

Outre les méthodes chimiques, il est possible d'utiliser des méthodes physiques pour enlever l'acide halohydrique, qui — si on le laisse dans le milieu réactionnel — risque de ralentir la réaction et de détruire le produit. Par exemple, on peut effectuer la réaction à une pression réduite de manière à aspirer l'acide, aussitôt qu'il est formé. Ceci peut être réalisé en utilisant un système présentant une interface importante,

tel qu'un évaporateur à film mince, pour faciliter le dégagement de l'acide. Il est également possible de faire passer un gas inerte, azote ou argon, par exemple, dans ou au-dessus du milieu réactionnel pour piéger l'acide dans le gaz et par conséquent l'enlever aussitôt qu'il se forme, ceci à pression relativement faible ou à la pression atmosphérique, voire à haute pression.

Il est également possible d'utiliser des tamis moléculaires, par exemple de 3 A° pour l'acide chlorhydrique.

Selon une autre variante de la présente invention, la déshydrohalogénation peut être effectuée en présence d'au moins un solvant aprotique — faiblement nucléophile ou non nucléophile — et, éventuellement, également d'un solvant polaire. Le solvant peut être choisi dans le groupe comprenant les éthers tels que le triglyme (diméthyléther de triéthylène glycol), les sulfones, les N,N-dialkylsulfonamides, les N,N,N',N'-tétralkylsulfonylurées, les hydrocarbures aromatiques et de préférence ceux parmi les hydrocarbures aromatiques qui ont un point d'ébullition approprié et au moins un substituant attracteur d'électrons (électrophiles), les alcanes et les alcènes ayant un point d'ébulliton approprié tels que le dichloroéthane et le tri- ou tétrachloroéthylène- et même le produit final, c'est-à-dire les carbamates déshydrohalogénes. Le chlorobenzène, le bromobenzène, les dichlorobenzènes, les trichlorobenzènes, les tétrachlorobenzène, les tétrachloroéthylène sont parmi les solvants les plus appropriés. L'utilisation des solvants simplifié fréquemment la récupération des produits. De préférence, on choisi les conditions de température et de pression de manière à opérer à reflux du solvant.

Une durée de quelques heures est habituellement suffisante pour obtenir les carbamates vinyliques. Il est préférable d'effectuer la réaction dans un milieu anhydre en l'absence d'oxygène, par exemple dans une atmosphère d'azote. Les carbamates vinyliques sont récupérés par des méthodes traditionnelles, par exemple après élimination du solvant dans des conditions appropriées; ou encore, s'ils sont relativement volatiles, ils peuvent être récupérées par distillation.

Il est possible et parfois commode de former le carbamate α-halogéné de départ et d'éliminer H—X pour obtenir le carbamate vinylique désiré sans isoler le carbamate α-halogéné intermédiaire. Par exemple, lorsque l'on utilise la chaleur dans la synthèse du carbamate α-halogéné la réaction peut fréquemment être effectuée de telle façon que l'élimination se produise presqu'aussitôt après que le carbamate halogéné est produit. En effet, une certaine quantité de carbamate α-halogéné peut subir l'élimination avant que le reste ait été complètement formé. Si l'élimination n'est pas complète dans ces conditions, le carbamate halogéné restant peut être transformé en carbamate vinylique dérivé en soumettant le mélange des deux substances aux conditions décrites dans cette demande pour convertir les carbamates α-halogénés pratiquement purs, de structure similaire, en carbamates vinyliques correspondants.

Selon une variante préférentielle du procédé, la réaction est effectuée en présence d'un catalyseur tel qu'il est décrit ci-dessus, en présence d'un accepteur organique de l'acide tel que décrit ci-dessus et en présence d'un solvant tel que décrit ci-dessus. D'excellents rendements sont fréquemment obtenus en utilisant le bromure de tétra-n-butyl-ammonium et la collidine en présence d'un solvant tel que le chlorobenzene ou le tétrachloroéthylène.

L'invention est illustrée par les exemples qui suivent.

Dans ces exemples, on utilise fréquemment l'abréviation ACE pour le groupe $CH_3$—CHCl—O—C(=O)—, ainsi que l'abréviation VOC pour le groupe $CH_2$—CH—O—C(=O)—. Ainsi, ACE—Cl désigne le chloroformiate d'α-chloroéthyle.

### Exemple 1

### Préparation de la N-Isobuténylоxycarbonyl-N-méthylcyclohexylamine

a) Synthése de la N-α-chloroisobutyloxycarbonyl-N-méthyl-cyclohexylamine

Une solution de N-méthylcyclohexylamine (Aldrich, séchée au-dessus de KOH et distillée) (14,9 g, 0,13 mole) dans 15 $cm^3$ d'éther a été ajoutée lentement (pendant 15 minutes) à une solution agitée de chloroformiate d'α-chloroisobutyle (9,91 g, 0,058 mol) dans 15 $cm^3$ d'éther refroidi à 0°C. Une fois l'addition achevée, le mélange a été chauffé à la température ambiante et agité encore pendant une heure. Les sels ont été éliminés par filtration et le filtrat a été rotoévaporé avant d'isoler le produit par simple distillation sous vide; on obtient 13,3 g (rendement de 92%) avec un point d'ébullition de 111 à 113°C à 80 Pa (0.6 mmHg).

IR* (μ): 3,49 (m), 5,81 (vs); $CCl_4$.

RMN $H^1$** (δ): 6,30 (d, J=4), 4,2—3,4 (m), 2,80 (s), 2,4—0,8 (m avec méthyle d à 1,05, J=6); rapport 1:1:3:17; $CCl_4$.

SM*** (m/e): 249, 1319 ($P^{37}Cl$, 6%, calc. 249,1310), 247,1344 ($P^{35}Cl$, 21%, calc. 247,1339), 206,0760 ($P^{37}Cl$—$CH(CH_3)_2$, 8%, calc. 206,0762), 204,0789 ($P^{35}Cl$—$CH(CH_3)_2$, 26%, calc. 204,0791), 157 (17%), 156 (88%), 140 (69%), 114 (68%), 58 (100%).

*IR Analyse par infra-rouge (m = moyen, vs = très fort, s = fort).

**RMN Analyse par résonnance magnétique nucléaire.

***SM Analyse par spectrométrie de masse.

b) Déshydrohalogénation

Le carbamate chloré (4,58 g, 0,0185 mole) a été chauffé par un bain d'huile à 170°C dans un ballon à

fond rond de 25 cm$^3$, équipé d'un réfrigérant à reflux et d'un tube d'introduction d'azote. On a noté un dégagement vigoureux de gaz HCl pendant les 30 premières minutes de la réaction; au bout d'une heure, le processus était achevé à 94% comme l'a montré l'analyse RMN). Au bout de 2 heures, le carbamate d'alcényle a été distillé directement à partir du mélange réactionnel. On a obtenu 3,29 g (rendement de 84%, pur selon RMN) avec un point d'ébullition de 98° à 102°C à 53 Pa (0,4 mmHg). On a retrouvé 0,46 g de résidu dans le ballon de distillation. Ce résidu a été analysé (RMN) comme constitué d'un mélange, à peu près dans la proportion de 1:1, d'hydrochlorure de N-méthylcyclohexylamine et du produit.

IR (µ): 3,48 (m), 5,84 (vs); $CCl_4$.

$^1$H NMR (δ): 7,0—6,7 (m), 4,3—3,5 (m), 2,83 (s), 2,2—0,5 (m); rapport 1:1:3:16; $CCl_4$.

MS (m/e): 211,1573 (P, 7%, calc. 211,1573), 140,1066 (P—OCH=$C(CH_3)_2$, 45%, calc. 140,1075), 83 (100%), 72 (42%).

c) N-α-isobuténlyloxycarbonyl-N-méthylcyclohexylamine

Une solution de N-α-chloroisobutyloxycarbonyl-N-méthyl-cyclohexylamine (10,1 g, 0,041 mole), 2,4,6-collidine (6,0 g, 0,050 mole), bromure de tétrabutylammonium (0,75 g, 0,002 mole) et tétrachloroéthylène (19 cm$^3$) a été chauffée à 125°C pendant 2 heures. Après une extraction exécutée de façon usuelle (éther et $H_2SO_4$ 1N et une rotoévaporation, le carbamate d'alcényle a été isolé sous forme pure (selon RMN) par distillation sous vide. On a obtenu 8,50 g (rendement de 99%) avec un point d'ébullition de 93 à 98°C à 26,6 Pa (0,2 mm Hg).

Exemple 2

Préparation de la N-vinyloxycarbonylpipéridine (VOC-pipéridine)

a) Préparation de la N-α-chloroéthoxycarbonylpipéridine (ACE-pipéridine)

On a fait tomber goutte à goutte une solution de N-éthylpipéridine (Aldrich, distillée) (5,68 g, 0,05 mole) dans 20 cm$^3$ de dichloroéthane, pendant 15 minutes, dans une solution agitée et réfrigérée de ACE—Cl (9,32 g, 0,065 mole), de 1,8-bis-(diméthylamino)-naphtalène (0,53 g, 0,0025 mole, 0,05 équiv.) et de 50 cm$^3$ de dichloroéthane. Au cours de l'addition, la couleur de la solution est passée de l'incolore au jaune et est restée jaune, pendant que le milieu réactionnel s'est refroidi. On a chauffé le mélange au reflux pendant 30 minutes, on l'a refroidi et l'on a enlevé sous vide le solvant et le ACE—Cl en excès. Ensuite le liquide rouge foncé restant a été distillé à 26,6 Pa (0,2 mmHg) pour fournir 9,36 g (rendement 97%) du carbamate attendu incolore; point d'ébullition 67° à 26,6 Pa (0,2 mm Hg).

IR (µ): 3,40 (m), 3,50 (m), 5,80 (vs), 7,01 (s); $CCl_4$.

$^1$H NMR (δ): 6,48 (q, J=6), 3,8 à 3,0 (mm), 1,9 à 1,2 (m, avec méthyle d à 1,75); rapport 1:4:9; $CCl_4$.

MS (m/e): 193,0653 (P[$^{37}$Cl], 4%, calc. 193,0684), 191,0700 (P[$^{35}$Cl], 11%, calc. 191,0700), 128,0712 (P[$^{35}$Cl]—$CHClCH_3$, 100%, calc. 128,0712), 112,0758 (P[$^{35}$Cl]—$OCHClCH_3$, 59%, calc. 112,0762), 84,0808 (73%).

b) Déshydrochloration

Dans un réacteur de verre équipé d'una agitateur magnétique, d'un thermomètre et d'un réfrigérant à reflux connecté avec un système à vide, on a introduit 9,8 grammes de N-α-chloroéthoxycarbonyl-pipéridine de formule:

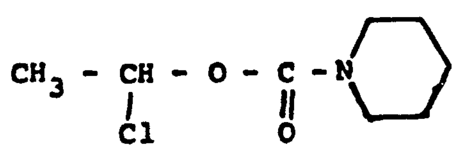

et 4,8 grammes (0,30 équivalent) de bromure de tétra-n-butyl-ammonium anhydre. On chauffe au reflux le mélange à une pression de 40 Pa (0.3 mm Hg) pendant une période de six jours (bain d'huile à 105°C). Le produit de réaction a ensuite été distillé sous pression réduite; on a ainsi obtenu de la VOC-pipéridine. Point d'ébullition: 60° à 61°C, à 40 Pa (0,3 mm Hg), rendement 78%. Dans l'analyse infra-rouge, le composé a présenté les bandes suivantes (µ): 5,81; 6,07; 8,57 dans $CCl_4$.

Exemple 3

Préparation de la N-vinyloxycarbonyl-pipéridine

Cette expérience a été effectuée de la même manière que celle décrite dans l'exemple 2, à partir des mêmes réactifs, mais ici on a chauffé au reflux le mélange à 40 Pa (0,3 mm Hg) (bain d'huile entre 85 et 95°C) pendant une période de 18 heures. Ensuite, on a ajouté du méthanol et on a chauffé au reflux le mélange pendant une heure, puis le méthanol a été enlevé sous pression réduite. L'huile restante a été extraite à l'éther et à l'eau. La couche d'éther a été évaporée pour obtenir la VOC-pipéridine pure avec un rendement de 54%.

Exemple 4

Préparation de la N-vinyloxycarbonyl-pipéridine

Dans un réacteur de verre sous vide, 0,55 gramme de chlorure de calcium a été séché pendant une

période d'une demi-heure. Ensuite, on a introduit la N-α-chloroéthoxycarbonyl-pipéridine en quantité de 0,75 $cm^3$. La température du ban d'huile a été maintenue à 130°C et le vide était de 5,3 kPa (40 mm Hg). Au bout d'une heure et quinze minutes, le rapport entre le produit et la matière de départ, mesuré par RMN, était 0:2:1. Au bout de vingt heures, la rapport, déterminé par RMN, entre le carbamate de vinyle et le carbamate de α-chloroéthyle était de 4,6.

Exemple 5

Préparation de la N-vinyloxycarbonyl-pipéridine

Cette expérience a été de la même manière que la précédente, mais le chlorure de calcium a été remplacé par du bromure de lithium. Le mélange a été chauffé pendant 1 heure et demie. Le rapport, déterminé par RMN, entre le carbamate de vinyle et le carbamate d'α-chloroéthyle était de 1:1.

Exemples 6—10

Préparation de la N-vinyloxycarbonyl-pipéridine

Ces expériences illustrent la déshydrochloration en présence de différents catalyseurs et différents solvants.

Dans ces exemples, la façon d'opérer a été la suivante:

Dans un réacteur, équipé d'un réfrigérant à reflux et d'un tube d'introduction d'azote, on introduit le solvant organique, la N-α-chloroéthoxycarbonyl-pipéridine et le catalyseur. Le mélange a été chauffé sous pression réduite, dans des conditions permettant un reflux modéré du solvant à la temperature de l'expérience. Les conditions expérimentales, les matériaux utilisés et les résultats sont indiqués au Tableau I. Les rendements indiqués ont été déterminés par spectroscopie RMN le mélange de réaction, à la fin de la durée de réaction citée.

TABLEAU I

| Ex. | N-$\alpha$-chloroethoxy-carbonyl-piperidine quantité en g | Catalyseur | Quantité en g. | Quantité en eq | Solvant | Temp. °C | Durée de réaction | Rendement VOC-Piperidine % |
|---|---|---|---|---|---|---|---|---|
| 6 | 0.92 | tetra-N-butyl-ammonium bromide | 0.16 | 0.10 | bromo-benzene 2 cc | 130 | 24 h | 78 |
| 7 | 0.92 | KCl + 18-couronne-6 | 0.18<br>0.13 | 0.5<br>0.10 | bromo-benzene 2 cc | 130 | 24 h | 53 |
| 8 | 0.97 | tetra-N-butyl-ammonium bromide | 0.09 | 0.055 | o-dichloro-benzene 2 cc | 150 | 20.5 h | 80 |
| 9 | 0.92 | tetra-N-butyl-ammonium bromide | 0.092 | 0.059 | triglyme 2 cc | 170 | 20 min | 55 |
| 10 | 7.190 | KCl + 18-couronne-6 | 0.96<br>0.47 | 0.35<br>0.05 | 1,2,4,6-tetra-chlorobenzene (28g) | 190 | 4.5 h | 61 |

Exemple 11

Préparation de la N-vinyloxycarbonyl-pipéridine (VOC-pipéridine) à partir de la ACE-pipéridine en présence de collidine

Une solution agitée (sous $N_2$) de ACE-pipéridine (9,6 g, 0,05 mole), collidine -2,4,6 (7,4 g, 0,06 mole, point d'ébullition 172°C) et o-dichlorobenzène (20 $cm^3$, 0,18 mole, point d'ébullition 178°C) a été chauffée à 185°C par un bain d'huile, laissé au reflux pendant trois heures et a ensuite été refroidie jusqu'à la température ambiante. Le mélange est devenu beaucoup plus foncé, et une certaine quantité d'hydrochlorure de collidine a précipité. Du chloroforme a été ajouté jusqu'à ce que le mélange soit homogène. Cette solution a été lavée avec de l'eau (3 × 50 $cm^3$) qui a enlevé presque tout la couleur, ensuite les couches aqueuses ont été combinées et extraites avec du chloroforme (2 × 30 $cm^3$). Les extraits de chloroforme ont été combinés, séchés ($Na_2SO_4$) et roto-évaporés. Le produit obtenu (la VOC-pipéridine) a été isolé au moyen d'une distillation efficace sous vide à travers une colonne chemisée à vide de 35 cm comportant un serpentin intérieur en téflon. La colonne était enveloppée d'un ruban chauffant maintenu à une température de 15 à 20°C plus basse que la température de distillation. La fraction d' o-dichlorobenzène a été suivie une fraction intermédiaire ayant son point d'ébullition à 95—123°C à 6,3 kPa (47 mm Hg) (2,07 g), fraction qui contenait (analyse RMN) du o-dichlorobenzène, de la collidine (3% mole) et de la VOC-pipéridine (31% mole, 9% de rendement absolu). La fraction finale de distillation, constitué de 6,21 g de produit ayant son point d'ébullition à 123—128°C à 6,3 kPa (47 mm hg), a été analysée par (RMN) comme étant de la VOC-pipéridine pure (rendement de 80%; produit total dans les deux fraction 89%). Presque aucun résidu n'a subsité dans le ballon de distillation.

Exemple 12

Préparation de la VOC-pipéridine

Dans une expérience similaire, du bromobenzène (20 $cm^3$, 0,19 mole, point d'ébullition 156°C) a été utilisé comme solvant-diluant inerte. La solution contenant le bromobenzène, la ACE-pipéridine (10,0 g, 0,052 mole) et la collidine (7,70 g, 0,064 mole) a été chauffée à 170°C par un bain d'huile, et l'on a laissé le mélange au reflux pendant 5,5 heures avant de le refroidir jusqu'à la température ambiante. Le mélange réactionnel a été extrait à l'eau (20 $cm^3$) et à l'éther (40 $cm^3$). Les couches ont été séparées, la couche organique a été lavée avec de l'eau (2 × 20 $cm^3$), et les extraits aqueux combinés ont été lavés avec de l'ether (2 × 25 $cm^3$). Les couches organiques ont été combinées, séchées ($Na_2SO_4$), rotoévaporées et distillées en utilisant l'appareillage décrit dans l'exemple 11. La fraction de bromobenzène a été suivie d'une petite fraction intermédiaire, 0,43 g, ayant son point d'ébullition à 100—124°C à 5,9 kPa (44 mm Hg) fraction qui a été analysée (RMN) comme étant un mélange de collidine (56% mole), de bromobenzène (27% mole) et de VOC pipéridine (17% mole). La fraction principale obtenue, soit 7,31 g de produit ayant son point d'ébullition à 124—126°C à 44 mm, a été analysée comme étant de la VOC-pipéridine contaminée par 3,5% moles de collidine-2,4,6 (rendement de VOC-pipéridine: 88%). La collidine a pu être éliminée par extraction à l'aide d'acide sulfurique aqueux dilué.

Exemple 13

Préparation de la VOC-pipéridine

Une autre expérience a été effectuée sur une échelle permettant l'analyse RMN, expérience où la collidine a été utilisée à la fois comme solvant et comme agent séquestrant d'acide. Dans ladite expérience, on a chauffé pendant une heure à 170°C, 1,12 g de ACE-pipéridine dans 3 $cm^3$ de collidine (3,9 équiv.). Dans l'analyse RMN, un rendement de 52% de VOC-pipéridine a été obtenu dans le milieu réactionnel et 33% de ACE-pipéridine initiale n'a pas transformé. Le reste du composé de départ a donné lieu à des produits de décomposition.

Exemple 14

Préparation de la VOC-pipéridine à partir de la ACE-pipéridine, en utilisant de la collidine et de la VOC-pipéridine additionnelle comme solvant-diluant

Une solution agitée (sous $N_2$) de ACE-pipéridine (8,33 grammes, 0,043 mole) collidine-2,4,6 (6,43 grammes, 0,053 mole) et VOC-pipéridine (15,6 g, 0,1 mole) a été chauffée à 185°C pendant 3 heures par un bain d'huile, puis refroidie et extraite avec l'éther (25 ml) et avec $H_2SO_4$ 1N (20 ml). Les couches ont été séparées et la couche organique a été lavée avec $H_2SO_4$ 1N additionnel (2 × 20 $cm^3$) et de la saumure (20 $cm^3$). Les couches acides aqueuses combinées ont été réextraites avec de l'ether (2 × 20 $cm^3$), que a été ensuite utilisé à réextraire la solution de saumure. Les extraits d'éther ont été combinés, séchés ($Na_2SO_4$), l'éther a été évaporé sous pression réduite et la VOC-pipéridine a été isolée par simple distillation sous vide; 20,6 g de composé ayant un point d'ébullition de 60—61°C à 40 Pa (0,3 mm hg) ont été obtenus (rendement après soustraction des 15,6 g inclus au début: 75%). Le produit était pur (analyse RMN). Un peu de résidue a subsisté dans le ballon de distillation.

Exemple 15

Préparation de la VOC-pipéridine

Dans un reacteur en verre, on a introduit 0,932 gramme de ACE-pipéridine, 0,098 gramme (0,063 équiv.) de bromure de tétra-n-butylammonium et 2 $cm^3$ (2,6 équiv.) de N,N'-diéthylaniline. Le mélange a

été chauffé à 150°C pendant 30 minutes. L'analyse (RMN) a montré que le produit était de la VOC-pipéridine suivant un rendement de 59%, et qu'une décomposition de 10% s'était produite. Le rapport de la VOC-pipéridine à la ACE-pipéridine était 1.9:1 (RMN).

Exemple 16

Préparation de la VOC-pipéridine à partir de la ACE-pipéridine en présence de collidine et de bromure de tétrabutylammonium

Une solution agitée (sous $N_2$) de ACE-piperidine (11,5 grammes, 0,06 mole), de bromure de tétrabutylammonium (0,97 gramme, 0,003 mole) et de collidine-2,4,6 (8,72 grammes, 0,07 mole) dans le chlorobenzène (25 $cm^3$, 0,25 mole, point d'ébullition 132°C) a été chauffée à 140°C dans un bain d'huile; puis on l'a laissée au reflux pendant 21 heures, et ensuite on l'a refroidie jusqu'à la température ambiante.

De l'éther (40 $cm^3$) a été ajoutée au mélange, qui a ensuite été extrait avec $H_2SO_4$1N (3 × 30 $cm^3$) et de la saumure (30 $cm^3$). Les couches aqueuses ont été combinées et réextraites avec de l'éther (2 × 40 $cm^3$). Les extraits à l'éther combinés ont été séchés ($Na_2SO_4$), rotoévaporés, et le chromobenzène a été chassé par distillation (point d'ébullition 55—57°C à 55 mm). La VOC-pipéridine a été isolée par distillation sous pression réduite; point d'ébullition 130—132°C à 55 mm; 8,2 grammes (rendement de 88%) pur selon RMN.

Dans une autre expérience effectuée dans le mêmes conditions — sauf que l'on a chauffé au reflux le mélange réactionnel pendant 28 heures — le rendement en VOC-pipéridine distillée était de 89%.

En dépit du fait que le catalyseur s'est décomposé dans les conditions de la réaction, celui-ci accroît néanmoins considérablement la vitesse de réaction, comme cela a été démontré par l'expérience suivante. De la ACE-pipéridine (10,4 grammes, 0,054 mole), de la collidine-2,4,6 (8,00 grammes, 0,066 mole) et du chlorobenzène (25 $cm^3$) ont été chauffés à 140°C pendant 27 heures. L'analyse par RMN a montré que la réaction était complète seulement à 84%. C'est pourquoi la réaction a été poursuivies à 140°C pendant une durée de réaction totale de 48 heures. L'opération ainsi décrite a fournir un rendement de 70% en VOC-pipéridine.

D'autres sels ont également pour propriété d'accroître la vitesse de réaction. Dans un autre expérience, un mélange de ACE-pipéridine (1,78 gramme, 9,3 moles) de collidine-2,4,6 (1,38 grammes, 11,4 mmoles), de chlorure d'ammonium (0,10 grammes, 1,9 mmoles) et de chlorobenzène (4 $cm^3$, 39,3 moles) a été chauffé à 140°C pendant 25 heures, et ensuite refroidi jusqu'a la température ambiante. De l'éther (20 $cm^3$) a été ajouté, et le mélange a été extrait avec $H_2SO_4$1N (2 × 20 $cm^3$). Les couches aqueuses combinées ont été relavées avec de l'éther (20 $cm^3$). Les couches organiques ont été combinées, séchées ($Na_2SO_4$) et rotoévaporées. Du tétrachloro-1,1,2,2 éthane (1,57 grammes, 9,35 moles) a été ajouté à l'huile restante, à titre d'étalon interne quantitatif en vue de l'analyse RMN; rendement calculé 63%.

Exemple 17

Préparation de la N,N'-di(vinyloxycarbonyl)-pipérazine (di-VOC-pipérazine)

a) N,N'-di(α-chloroéthoxycarbonyl)-pipérazine (di-ACE-pipérazine)

Une solution de N,N'-diméthyl-pipérazine (Aldrich, séchée au-dessus de KOH et obtenu par distillation fractionnée) (9,50 g, 0,083 mole) dans du 1,2-dichloroéthane (40 ml) a été versée goutte à goutte (pendant 35 minutes) dans une solution agitée de chloroformiate d,α-chloroéthyle (32,5 g, 0,227 mole) et de bis(diméthylamino)-1,8-naphtalène (1,94 g, 0,009 mole, 0,11 équiv.) dans le dichloroéthane (100 $cm^3$) maintenu à −5°C. Pendant l'addition de la pipérazine, un solide blanc a précipité, ce qui a fait décroître l'efficacité de mélange, mais lorsque la température de réaction a été augmentée jusqu'au reflux, le solide s'est dissout. Après 1 heure de reflux, la solution de teinte rouge-marron a été refroidie, du charbon de bois a été ajouté, on a fait passer doucement du HCl sec à travers la solution pendant 30 secondes pour complexer le bis (diméthylamino)-naphtalène en excés, et l'on a fait passer la solution à travers un tampon de gel de silice de dimensions 1,9 × 5,08 cm (3/4" × 2") avec due chlorure de méthylène comme éluant. L'évaporation sous vide de la solution limpide obtenue a donné lieu à une solution d'un blanc légèrement teinté, solution qui a été séchée pendant une nuit sous vide à 55°C; le rendement a été de 97% (24,0 g, pur selon RMN) de dicarbamate attendu, ayant un point de fusion de 125,5—135,5°C (la plus grande partie du solide a fondu à 125,5—130°C). Le solide a eu son point de fusion à 131,5—138°C après recristallisation dans du 1,2-dichloroéthane, mais sa pureté spectrale et CCM (chromographie couche mince restait inchangée. Puisque le solide est probablement un mélange d'une paire de diastéréoisomères, le point de fusion ne fournit pas une bonne indication de la pureté, mais seulement une indication de la variation du rapport des diastéréoisomères. Les deux centres de chiralité sont toutefois trop éloignés entre eux pour démontrer la présence de diastéréoisomères dans le spectre RMN.

IR (μ): 5,79 (vs), 8,17 (m), 9,17 (s); $CHCl_2$.

$^1$H NMR (δ): 6,58 (q, J=6), 3,52 (large d), 1,79 (d, J=6); rapport 2:8:6; $CDCl_3$.

MS (m/e): 300,0447 ($P^{37}Cl$, $^{35}Cl$, 2%, calc. 300,0457), 298,0471 ($P^{35}Cl_2$, 4%, calc. 298,0487), 237 (7%), 235,0476 ($P^{35}Cl$ —$CHClCH_3$, 19%, calc. 235,0485), 221 (7%), 219,0530 ($P^{35}Cl$ —$OCHClCH_3$, 12%, calc. 219,0536), 191 (29%), 177 (34%), 175 (100%), 155 (43%), 149 (66%), 113 (32%).

b) Conversion de la N,N'-di-ACE-pipérazine en N,N'-di-VOC-pipérazine

Une solution de N,N'-di-ACE-pipérazine (7,61 g, 0,025 mole), de collidine-2,4,6 (6,96 g, 0,057 mole) et de o-dichlorobenzène (20 $cm^3$) a été chauffée (par un bain d'huile à 185°C) pendant 45 minutes. Du

chloroforme (40 $cm^3$) a été ajouté à la solution refroidie, de teinte rouge foncée, qui a ensuite été extraite avec $H_2SO_4$ 1N (3 × 30 $cm^3$) et de la saumure (20 $cm^3$). Les extraits aqueux ont été réextraits avec du chloroforme (2 × 25 $cm^3$). Les couches organiques combinées ont été séchées ($Na_2SO_4$), du charbon de bois a été ajouté, et le solvant a été enlevé sous vide. On a fait passer le résidu solide à travers un tampon en gel de silice (en utilisant $CH_2Cl_2$ comme éluant). L'éluat a été évaporé sous pression réduite, pour donner la N,N'-di-VOC-pipérazine sous forme de solide teinte légèrement tannée; la quantité obtenue était la 1,88 g (rendement de 33%, RMN pur). L'échantillon rechromatographié, utilisé pour l'analyse, avait un point de fusion de 97,5 à 99°C.

IR (μ): 5,86 (vs), 6,06 (m); $CH_2Cl_2$.

$^1$H NMR (δ): 7,21 (d de d, J=14, 6), 4,80 (d de d, J=14, 1), 4,48 (d de d, J=6, 1), 3,54 (s); rapport 2:2:2:8; $CDCl_3$.

MS (me): 226,0947 (P, 15%, calc. 226,0953) 183,0762 (P—OCH=$CH_2$, 98%, calc. 183,0770), 139 (42%), 113 (100%), 97 (28%).

Dans une expérience similaire, effectuée dans le bromobenzène (6,6 équiv.) au reflux, en présence de 2,3 équiv. de collidine-2,4,6 la réaction était complète à 42% (analyse RMN) après 2 heures.

Dans une autre expérience, une solution de N,N'-di-ACE-pipérazine (5,15 g, 0,017 mole), de bromure de tétra-n-butylammonium (0,51 g, 0,002 mole), de collidine-2,4,6 (4,91 g, 0,041 mole) de tétrachloroéthylène (18 $cm^3$) a été chauffé au reflux. Après 3 heures, l'analyse RMN indiquait que la réaction était complète à 28%. Le mélange réactionnel a été traité, suivant la description donnée ci-dessus, au bout de 24 heures, et la N,N'-di-VOC-pipérazine a été isolée.

La réaction de la N,N'-di-ACE-pipérazine (1,71 g, 5,72 mmoles) dans le dichloroéthane (20 $cm^3$) en présence de chlorure de benzyltri-n-butylammonium (1,30 g, 4,15 mmoles) a été effectuée au reflux, pendant une nuit, en faisant passer du $N_2$ à travers la solution et en laissant le solvant s'évaporer. L'huile restante, de teinte marron, s'est solidifiée à la suite du refroidissement. Elle a été extraite par $CH_2Cl_2$ (50 $cm^3$) et HCl 0,01 N (40 $cm^3$). La couche organique a été séchée ($Na_2SO_2$), evaporée, puis chauffée à 65°C dans 5 $cm^3$ de méthanol. Après évaporation et une tentative de sublimation, le produit a été chromatographié à travers le siliçe. Le rendement de N,N'-di-VOC-pipérazine chromatographiée a été de 33%.

De la N,N'-di-VOC-pipérazine a également été produite en l'absence de catalyseur, de solvant ou de base. Lorsque la N,N'-di-ACE-pipérazine (0,62 g, 2,07 mmoles) a été chauffée à 185°C sous un vide de 100 mm, pour enlever HCl formée, pendant 1 heure, l'analyse RMN a indiqué une conversion de 34% en VOC et une décomposition de 10% composé initial.

Dans une dernière expérience, du (−)-β-pinène a été utilisé pour piéger HCl. On a chauffé au reflux la N,N'-di-ACE-pipérazine (10,1 g, 0,034 mole), le (−)-β-pinène (11,1 g, 0,082 mole) et le o-dichlorobenzène (25 $cm^3$), par un bain d'huile à 185°C. Aprés 20 heures, l'analyse RMN a montré que la réaction était achevée à 70%. Au bout 41 heures, le solvant a été enlevé sous pression réduite, du charbon de bois a été ajouté, et l'on a fait passer le produit à travers un tampon en gel de silice (en prenant du $CH_2Cl_2$ comme éluant), L'analyse RMN du produit chromatographié (4,29 g) a indiqué un rapport VOC/ACE de 86 à 14; rendement absolu an di-VOC pipérazine: 40%.

Exemple 18

Préparation de la N-vinyloxycarbonyl-N-méthylaniline (N-VOC-N-méthylaniline

a) Préparation de la N-ACE-N-méthylaniline

De la N,N-diméthylaniline (19,3 grammes, 0,16 mole) dans du dichloroéthane (25 $cm^3$) a été ajoutée lentement a une solution agitée, refroidie (à 0°C) de ACE-Cl (49,8 grammes, 0,35 mole) dans le dichloroéthane (75 $cm^3$). Ce mélange a été chauffé à 90°C (bain d'huile), puis on l'a chauffé au reflux pendant 3 jours. Le solvant et le ACE-Cl en excès ont été enlevés par évaporation sous vide. Une huile noire a été obtenue, à laquelle on a ajouté de l'éther (qui a eu pour effet de précipiter une matière solide noire) et du carbon de bois. Les solides ont été éliminés par filtration, le solvant a été enlevé, et le produit a été isolé par distillation sous vide; 29,3 grammes (rendement de 86%, pur selon RMN) ont été obtenus, avec le point d'ebullition à 100—110°C à 53 Pa (0.4 mm Hg).

IR (μ): 5,78 (vs), 6,26 (m), 6,67 (m); $CCl_4$.

$^1$H NMR (δ): 7,6—6,9 (m), 6,55 (q, J=6), 3,21 (s), 1,60 (d, J=6); rapport 5:1:3:3; $CCl_4$.

MS (m/e): 215,0562 ($P^{37}Cl$, 38%, calc. 215,0527), 213,0573 ($P^{35}Cl$, 71%, calc. 213,0556), 151,0646 (P—$C_2H_3Cl$, 64%, calc. 151,0634), 134,0594 (P—$OCHClCH_3$, 100%, calc. 134,0606), 107 (64%), 106 (76%), 77 (69%), 63 (80%).

b) conversion facilitée par la collidine, de la N-ACE-N-méthylaniline en N-VOC-N-méthylaniline

Un mélange agité (sous $N_2$) de N-ACE-N-méthylaniline (10,4 grammes, 0,048 mole), de collidine-2,4,6 (7,15 grammes, 0,059 mole) et de o-dichlorobenzène (20 $cm^3$, 0,18 mole) a été chauffé à 185°C pendant 3 heures par un bain d'huile, puis refroid à la température ambiante. Le mélange a été extrait à l'eau et à l'éther, les couches ont été séparées, la couche organique a été lavée avec de l'eau, séchée (avec du $Na_2SO_4$) et évaporée sous pression réduite. La N-VOC-N-méthylaniline obtenue a été isolée par distillation sous vide à travers une courte colonne de Vigreaux; on a obtenu 2,72 grammes (rendement de 32%, pur selon RMN) de produit ayant son point d'ébullition à 75—79°C à 53 Pa (0,4 mm Hg).

IR (μ): 5,78 (vs), 6,06 (m), 6,25 (m), 6,67 (m); $CCl_4$.

$^1H$ NMR (δ): 7,5—6,8 (M), 4,57 (d de d, J=14, 1), 4,30 (d de d, J=7, 1), 3,20 (s); rapport 6:1:1:3; $CCl_4$.

MS (m/e): 177,0793 (P, 58%, calc. 177,0790), 134,0608 (P—OCH=$CH_2$, 100%, calc. 134,0606), 1119,0371 (P—$C_3H_6O$, 12%, calc. 119,0371), 106,0655 (P—$CO_2$—CH=$CH_2$, 81%, calc. 106,0657), 77,0391 ($C_6H_5$, 81%, calc. 77,0391), 51 (28%).

Exemple 19

Preparation of de la N-vinyloxycarbonyl-morpholine

a) Préparation de la N-α-chloroéthoxycarbonyl-morpholine (ACE-morpholine)

Un échantillon de N-méthyl-morpholine a été transformé en N-α-chloroéthoxycarbonyl-morpholine avec un rendement de 96% par réaction, de manière analogue à ce qui décrit dans l'exemple 18; point d'ébullition 84—86°C à 53 Pa (0,4 mm Hg).

IR (μ): 5,79 (vs), 7,82 (m), 8,08 (s), 9,11 (s); $CCl_4$.

$^1H$ NMR (δ): 6,50 (q, J=6), 3,8—3,2 (m), 1,76 (d, J=6); rapport 1:8:3; $CCl_4$.

MS (m/e): 195,0462 (P[$^{37}$Cl], 3%, calc. 195,0476), 193,0494 (P[$^{35}$Cl], 8%, calc. 193,0506), 180,0237 (P[$^{37}$Cl]—$CH_3$, 6%, calc. 180,0241), 178,0269 (P[$^{35}$Cl]—$CH_3$, 19%, calc. 178,0271), 130,0500 (P—CHCl$CH_3$, 39%, calc. 130,0504), 114,0550 (P—OCHCl$CH_3$, 71%, calc. 114,0555).

b) Conversion de la ACE-morpholine en VOC-morpholine

Un mélange agité de ACE-morpholine (11,3 grammes, 0,058 mole) et de collidine-2,4,6 (8,79 grammes, 0,073 mole) dans le o-dichlorobenzène (25 $cm^3$, 0,22 mole) a été chauffé à 185°C pendant 2 heures par un bain d'huile (une analyse RMN, effectuée au bout d'une heure et demie, a montré que la réaction était achevée à 96%). Après le traitement normal d'extraction à l'éther $H_2SO_4$ 1N et la rotoévaporation, le produit a été isolé par distillation sous vide à travers une colonne de 35 cm chemisée comportant un serpentin intérieur en téflon. Le solvant a été enlevé (point d'ébullition 93—94°C à 6,5 kPa (49 mm Hg); il a été suivi d'une fraction qui contenait 9% mole de VOC-morpholine (1,90 grammes, point d'ébullition 109—126°C à 6,5 kPa (49 mm Hg), rendement calculé 2%).

Par distillation on a obtenu 7,32 grammes de produit (rendement de 80%, pur selon RMN) ayant son point d'ébullition à 134—136°C à 49 mm (rendement global 82%).

IR (μ): 5,81 (vs), 6,07 (m); $CCl_4$.

$^1H$ NMR (δ): 7,20 (d de d, J=14, 7), 4,68 (d de d, J=14, 1) et 4,40 (d de d, J=7, 1), 3,9—3,2 (large); rapport 1:2:8; $CCl_4$.

MS (m/e): 157,0732 (P, 14%, calc. 157,0739), 114,0563 (P—OCH=$CH_2$, 88%, calc. 114,0555), 70 (100%).

Exemple 20

Preparation de l'(isobuténylоxycarbonyl)-1-benzotriazole

a) (α-chloroisobutyloxycarbonyl)1-benzotriazole

Du chloroformiate d'α-chloroisobutyle (7,85 grammes, 0,046 mole) dans l'éther (10 $cm^3$) a été ajouté (pendant 10 minutes) à une solution agitée, refroidie (0°C) de benzotriazole (Aldrich) (11,6 grammes, 0,097 moles), d'éther (50 $cm^3$) et de chlorure de méthylène (10 $cm^3$). Une fois l'addition achevée (au bout de 10 minutes), le mélange a été agité pendant 1 heure à la température ambiante. Le benzotriazole en excés a été enlevé en faisant passer du HCl dans le mélange, puis en enlevant tous les sels par filtration. Ensuite, on a fait passer le produit à travers un tampon en gel de silice (le $CH_2Cl_2$ servant d'éluant). L'huile limpide obtenue après rotoévaporation du solvant (11,5 grammes, rendement de 99%) s'est ensuite solidifiée (point de fusion 42—52°C).

IR (μ): 5,65 (vs), 6,19 (w), 6,23 (w), 6,71 (m), 6,86 (vs), 7,10 (vs); $CCl_4$.

$^1H$ NMR (δ): 8,3—7,3 (m), 6.63 (d, J=5), 2,9—2,2 (m), 1,22 (d, J=6); rapport 4:1:1:6; $CDCl_3$.

MS (m/e): 255,0582 (P[$^{37}$Cl], 6%, calc. 255,0588), 253,0626 (P[$^{35}$Cl], 18%, calc. 253,0618), 146 (85%), 135 (20%), 119 (37%), 118 (43%), 91 (67%), 90 (92%), 55 (100%).

b) Conversion de l'(α-chloroisobutyloxycarbonyl)-1-benzotriazole en (isobuténylоxycarbonyl)-1-benzotriazole

Le chloroisobutyloxycarbonyl-benzotriazole (4,72 grammes, 0,019 mole) et le bromure de tétrabutylammonium (0,029 grammes, 0,001 mole) ont été séchés pendant nuit sous vide à 50°C. Du tétrachloroéthylène (10 $cm^3$) et de la collidine-2,4,6 (2,83 grammes, 0,023 mole) ont été ajoutés, puis on a chauffé au reflux le mélange (en utilisant un bain d'huile à 125°C) pendant 9 heures (d'après l'analyse RMN, la réaction était achevée à 70% après 3 heures). La solution, de teinte rouge foncée, a été passé à travers un tampon en gel de silice (en prenant du $CH_2Cl_2$ comme éluant). L'huile rouge subsistant après la rotoévaporation a été passé à travers un autre tampon de silice élué avec de l'éther: hexane (1:1), la rotoévaporation a fourni le produit sous forme d'huile jaune (2,48 grammes, 61% de rendement brut). La cristallisation dans l'hexane a donné lieu à une matière solide jaune (1,81 grammes, pur à plus de 95% d'après l'analyse RMN, 43% de rendement). Le filtrat contenait également environ 50% de produit (analyse RMN) (rendement total 50%). Le produit a été recristallisé à nouveau à partir de l'hexane; le point de fusion a été de 47 à 49°C. Martz (Ph. D. Thesis, The Pennsylvania State University, U.S.A., 1982) a rapporté un point de fusion de 47°—49,5°C pour le produit obtenu en traitant le fluorure de benzotriazole-1-carbonyle avec de l'isobutyloxytriméthyl-silane. Les propriétés spectrales citées ci-après concordent avec celles données par

Martz (loc. cit.).

IR (µ): 5,68 (vs), 6,21 (w), 6,24 (w), 6,72 (s), 6,89 (s), 7,15 (vs); $CHCl_2$.

$^1H$ NMR (δ): 8,3—7,2 (m), 7,2—6,9 (m), 1,90 (s), 1,76 (s); rapport 4:1:3:3, $CDCl_3$.

Lorsque la réaction a été effectuée dans l'o-dichlorobenzène à 170°C pendant 1 heure (pas de $(nBu)_4N^+ Br^-$ présent), le rendement (suivant l'analyse RMN) a été de 30%; une grande quantité de composé de départ et de produits de décomposition était également présente. Lorsque le chloroisobutyloxycarbonyl-benzotriazole a été chauffé à 170°C sous vide à 21,3 kPa (160 mm Hg) pendant 2 heures, le produit a été formé avec un rendement de 10% (analyse RMN) et le reste du mélange était constitué presque exclusivement du composé de départ.

## Exemple 21

### Préparation de la N(E,Z-propényloxycarbonyl-morpholine

a) N-(α-chloropropyloxycarbonyl)-morpholine

Une solution de morpholine (Aldrich, séchée au-dessus de KOH et distillée) 16,1 grammes, 0,18 mole) dans l'éther (25 $cm^3$) a été ajoutée (pendant 15 minutes) à une solution agitée, refroidie (0°C) de 1-chloropropyl-chloroformiate (13,0 grammes, 0,083 mole) dans l'éther (25 $cm^3$). Le mélange a été agité pendant 1 heure à la température ambiante, les sels ont été éliminés par filtration, le filtrat a été concentré, et le produit distillé: point d'ébullition 95—97°C à 120 Pa (0,9 mm Hg), 14,4 grammes (rendement de 84%).

IR (µ): 5,81 (vs); $CCl_4$.

$^1H$ NMR (δ): 6,37 (t, J=6), 3,9—3,2 (large), 2,02 (pentet, J=6,7), 1,02 (t, J=7); rapport 1:8:2:3; $CDCl_3$.

MS (m/e): 209,0636 ($P[^{37}Cl]$, 6%, calc. 209,0633), 207,0662 ($P[^{35}Cl]$, 20%, calc. 207,0662), 130,0503 (P—CHClEt, 27%, calc. 130,0504), 114,0562 (P—OCHClEt, 81%, calc. 114,0555), 41 (100%).

b) Conversion de la N-(1-chloropropyloxycarbonyl)-morpholine en N-(E,Z-propényloxycarbonyl)-morpholine

Un mélange agité de 1-chloropropyloxycarbonyl-morpholine (11,9 grammes, 0,057 moles), de collidine-2,4,6 (8,6 grammes, 0.071 mole), de bromure de n-tétrabutylammonium (0,94 grammes, 0,003 mole) et de tétrachloroéthylène (25 $cm^3$) a été chauffé à 125°C par un bain d'huile pendant 24 heures (suivant l'analyse RMN, la réaction était achevée à environ 70% aprés 5 heures). Aprés le traitement usuel d'extraction, l'éther et les autres substances volatiles ont été chassés par distillation, et le produit a été isolé par distillation sous vide; point d'ébullition 86°—90°C à 66,6 Pa (0,5 mm Hg), 8,2 grammes (rendement de 84%). Les propriétés spectrals indiquées ci-dessus concordent avec celles rapportées par Olofson et Cuomo, *Tetrahedron Letters, 21,* 819 (1980). Sur la base des analyses spectrales et des données d'Olofson et Cuomo par les isomères purs, le rapport E:Z pour le nouveau produit a été déterminé comme étant de 1:1,8.

IR (µ): 5,81 (vs), 5.95 (w); $CCl_4$.

$^1H$ NMR (δ): 7,2—6,8 (m), 5,19 (d de q, J=1,5, 7, isomère E), 4,73 (de de q, J=7, 7, isomère Z), 3,9—3,2 (large), 1,8—1,5 (m); rapport 1:0,36:0,64:8:3; $CCl_4$.

Lorsque la déshydrochloration a été effectuée dans la bromobenzène (bain d'huile à 170°C sans le bromure de tétrabutylammonium) pendant 90 minutes, le rendement estimé en produit (suivant RMN) était de 55% (E:Z 1:2); on a retrouvé environ 25% du produit de départ, le reste étant représenté par les produits de décomposition.

## Exemple 22

### Préparation of de la N-vinyloxycarbonyl-N-méthylcyclohexylamine

a) Préparation de la N-α-chloroéthoxycarbonyl-N-méthyl-cyclohexylamine

Une solution de N-méthyl-cyclohexylamine (22,1 g, 0,2 mole) dans l'éther (10 $cm^3$) a été ajoutée (pendant 20 minutes) à une solution agitée, refroidie (à 0°C) de chloroformiate d'α-chloroéthyle (ACE-Cl) (28,3 g, 0,2 mole) dans l'éther (25 $cm^3$). Le mélange a été agité encore pendant une heure à la température ambiante, puis les solides ont été séparés par filtration. L'évaporation du filtrat a été suivie d'une distillation sous vide; 19,9 g (rendement de 93%) de produit ont été obtenus, avec un point d'ébullition de 101—103°C à 66,6 Pa (0,5 mm Hg).

IR (µ): 5,82 (vs); $CCl_4$.

$^1H$ NMR (δ): 6,55 (q, J=6), 4,2—3,5 (m), 2,80 (s), 2,2—0,9 (m avec d à 1,80); rapport 1:1:3:13; $CCl_4$.

MS (m/e): 221,0995 ($P[^{37}Cl]$, 5%, calc. 221,0997), 219,1024 ($P[^{35}Cl]$, 16%, calc. 219,1026), 156,1028 ($P—CHClCH_3$, 93%, calc. 156,1024), 114 (73%), 70 (68%), 63 (94%), 55 (74%), 42 (100%).

b) Conversion, par déshydrohalogénation, en N-VOC-N-méthylcyclohexylamine

Une solution agitée de N-ACE-N-méthylcyclohexylamine (5,49 g, 0,025 mole) préparée suivant la description donnée dans l'ex. 22 a), de collidine-2,4,6 (4,97 g, 0,041 mole) et de bromobenzène (12 $cm^3$) a été chauffé (bain d'huile à 170°C) pendant 1,5 heures (dans une réaction antérieure à l'échelle analytique, le produit a été reformé avec un rendement de 90%, et 10% de produits de décomposition étaient présents au bout de 2 heures, d'après l'analyse RMN). Après le traitement usuel d'extraction, le produit a été isolé par distillation sous vide; son point d'ébullition se situait à 81—85°C à 26,6 Pa (0,2 mm Hg) alors que R. C. Schnur, Ph. D. Thesis, The Pennsylvania State University, U.S.A., 1973, rapportait un point d'ébullition de

119°C à 1,9 kPa (14 mm Hg). Le produit (4,09 g) était contaminé par environ 12% de matière premiére (rendement absolu corrigé 78%, suivent l'analyse RMN).

IR (μ): 5,84 (vs), 6,06 (m); $CCl_4$.

$^1$H NMR (δ): 7,19 (d de d, J=14, 6), 4,65 (d de d, J=14, 1), 4,32 (de de d, J=6, 1), 4,2—3,6 (m), 2,81 (s), 2,1—0,8 (m); rapport 1:1:1:1:3:10; $CCl_4$.

Dans une autre expérience, une solution agitée de N-ACE-N-méthylcyclohexylamine (10,4 g, 0,047 mole), de (−)-β-pinène (8,1 g, 0,06 mole, point d'ébullition 165—167°C) et d'o-di-chlorobenzène (25 $cm^3$) a été chauffé (bain d'huile à 85°C) pendant 6 heures. Une distillation fractionnée sous vide a donné 3,44 g de N-VOC-N-méthylcyclohexylamine (rendement de 40%; pur selon RMN) ayant un point d'ébullition de 90—92°C à 80 Pa (0,6 mm Hg). Le résidu subsistant dans le ballon de distillation a été identifié (par RMN) comme étant du chlorhydrate de N-méthylcyclohexylamine (env. 58% récupérés).

Dans un essai du β-pinène à la fois comme solvant et comme agent accepteur d'acide, une solution de N-ACE-N-méthylcyclohexylamine dans le (−)-β-pinène (rapport 1:4,6 équiv.) a été chauffé à 170°C pendant 1,5 heures. Par analyse RMN (tétrachloro-1,1,2,2 éthane comme étalon interne quantitatif), le mélange a été identifié comme constitué de 64% de composé de départ résiduel et 43% de produit VOC.

Exemple 23

Péparation de N,N-diéthyl-O-vinyl-carbamate (VOC-diéthylamine)

a) Synthèse du N,N-diéthyl-O-α-chloroéthyl-carbamate

De la triéthylamine (17,6 g, 0,17 mole) dans 25 $cm^3$ de dichlorométhane a été ajoutée lentement à une solution agitée, refroidie (à 0°C) de ACE-Cl (30,3 g, 0,21 mole) dans le dichloroéthane (75 $cm^3$). On a chauffé au reflex la solution pendant une heure, après quoi le solvant a été enlevé et le produit isolé par distillation sous vide; 29,8 g (rendement de 96%, pur selon RMN) de produit ont été obtenus, avec un point d'ébullition de 83 à 89°C à 1,5 kPa (11 mm Hg).

IR (μ): 5,81 (vs); caractérisé.

$^1$H NMR (δ): 6,58 (q, J=6), 3,30 (q, J=7), 1,80 (d, J=6), 1,13 (t, J=7); rapport 1:4:3:6; $CCl_4$.

MS (m/e): 181,0688 (P[$^{37}$Cl], 7%, calc. 181,0683), 179,0712 (P[$^{35}$Cl], 21%, calc. 179,0713), 166,0436 (P[$^{37}$Cl]—Me, 13% calc. 166,0488), 164,0471 (P[$^{35}$Cl]—Me, 41%, calc. 164,0478), 116,0709 (P—$CHClCH_3$, 30%, calc. 116,0711), 102,0551 (P—$C_3H_6Cl$, 39%, calc. 102,0555), 100,0764 (P—$OCHClCH_3$, 96%, calc. 100,0763), 72,0816 ($Et_2N$, 50%, calc. 72,0813), 65 (32%), 63 (100%), 58 (82%).

b) N,N-diéthyl-O-α-bromoéthyl-carbamate

C'est l'analogue bromé du carbamate d'α-chloroéthyle dont il est question plus haut; ce produit a présenté un point d'ébullition de 63—66°C à 93,3 Pa (0,7 mm Hg) ainsi que les données spectrales indiquées ci-dessous.

IR (μ): 5,78 (vs); $CCl_4$

$^1$H NMR (δ): 6,69 (q, J = 6), 3,24 (q, J = 7), 1,97 (d, J = 6), 1,12 (t, J = 7); rapport 1:4:3:6; $CCl_4$

MS (m/e): 225,0227 (P[$^{81}$Br], 3%, calc. 225,0188), 223,0214 (P[$^{79}$Br], 3%, calc. 223,0208), 166 (11%), 164 (11%), 144 (18%), 109 (40%), 107 (39%), 101 (19%), 100 (100%), 72 (86%).

c) Déshydrohalogénation

1. Conversion du carbamate de N,N-diéthyl-O-α-bromoéthyle en VOC-diéthylamine

Un bain d'huile contenant un flacon avec une solution agitée de $CH_3CHBrOC(=O)NEt_2$ (9,6 g, 0,043 mole), de collidine-2,4,6 (6,6 g, 0,055 mole), de bromure de tétrabutylammonium (0,73 g, 0,002 mole) et de tétrachloroéthylène (18 $cm^3$, point d'ébullition 121°C) a été chauffé à 125°C pendant 1,5 heures (suivant l'analyse RMN, la réaction était en fait terminée au bout d'une heure environ). Après le traitement usuel d'extraction et l'élimination des substances volatiles par distillation, le produit (VOC—$NEt_2$) a été isolé par distillation sous pression réduite; on a obtenu 5,6 g de produit (rendement de 92%, pur selon RMN) avec un point d'ébullition de 97—98°C à 8,3 kPa (62 mm Hg). Les propriétés spectrales du produit concordent avec celles rapportées par R.C. Schnur (Ph. D. Thesis, The Pennsylvania State University, U.S.A., 1973). Schnur a rapporté un point d'ébullition de 63°C à 1,7 kPa (13 mm Hg).

$^1$H NMR (δ): 7,20 (d de d, J = 7, 14), 4,65 (d de d, J = 14, 1), 4,31 (d de d, J = 7, 1), 3,29 (q, J = 7), 1,12 (t, J = 7); rapport 1:1:1:1:4:6; $CCl_4$

Dans une autre expérience, une solution du même carbamate de bromoéthyle (12,7 g, 0,057 mole), de collidine-2,4,6 (8,5 g, 0,070 mole), de chlorure de tétra-n-hexylammonium (1,25 g, 0,003 mole) et de trichloroéthylène (23 $cm^3$) a été réchauffée à 95°C pendant 5 heures (la réaction était achevée à 90% au bout de 2 heures). La fraction de distillation du produit (pur selon RMN) contenait 3,81 g (rendement de 47%) ayant un point d'ébullition à 91—93°C à 6,9 kPa (52 mm Hg).

Dans une expérience réalisée dans le dichloro-1,2 éthane à 95°C et utilisant la collidine et le $Bu_4N^+Br$ comme promoteurs, le rendement estimé (RMN) de VOC—$NEt_2$, était de 26% au bout de 2 heures et demie.

2. Convension du carbarmate, de N,N-diéthyl-O-α-chloroéthyle en VOC-diéthylamine

La réaction du $CH_3CHClOC(=O)NEt_2$, était plus lente que celle provenant du composé α-bromé, comme le montre l'expérence suivante. Lorsque le AEC—$NEt_2$ remplaçait le correspondant bromé dans la première expérience de cette série, seulement 15% de VOC—$NEt_2$ ont été formés au bout de 2 heures à 125°C dans le tétrachloroéthylène, et il est resté 85% du ACE—$NEt_2$ de départ (analyse RMN). En allongeant les durées de

réaction, toutefois, le rendement a été augmenté.

Exemple 24

Préparation du carbamate de N,N-diméthyl-O-vinyle (VOC-diméthylamine)

a) Préparation de l'ACE-diméthylamine.

Le composé a été préparé par N-déméthylation de la triméthylamine avec ACE—Cl dans le dichloroéthane; point d'ébullition 82 à 84°C à 2,3 kPa (17 mm Hg).

IR (μ): 5,81 (vs); $CCl_4$

$^1H$ NMR (δ): 6,57 (q, J = 6), 2,93 (s), 1,80 (d, J = 6), rapport 1:6:3; $CCl_4$

MS (m/e): 153,0379 (P[$^{37}Cl$], 3%, calc. 153, 0370), 151,0399 (P[$^{35}Cl$], 11%, calc. 151,0400), 89 (17%), 88 (20%), 72,0447 (P—$OCHClCH_3$, 100%, calc. 72,0449), 65 (12%), 63 (38%).

b) Conversion de l'ACE-diméthylamine en VOC-diméthylamine

Une solution de ACE-diméthylamine (7,84 g, 0,052 mole), de collidine-2,4,6 (7,54 g, 0,062 mole) et de 20 $cm^3$ de N,N,N',N'-tétraéthylsufamide ($Et_2NSO_2NEt_2$, séché au-dessus de tamis 4 Å) a été chauffée à 155°C pendant 30 minutes, lorsque l'analyse RMN a indiqué que la réaction était achevée à 80%. Le produit, à bas point d'ébullition, a été séparé du solvant et des sels, à haut point d'ébullition, par distillation sous vide. La fraction ayant son point d'ébullition de 66—71°C à 4 kPa à 30 mm Hg) contenait du VOC—$Me_2$ avec un rendement absolu de 32%, contaminé par de la collidine et un peu de ACE—$NMe_2$ et de $Et_2NSO_2NEt_2$. Les impuretés ont été enlevées en chauffant au reflux le produit, avec 10 cm de MeOH, pendant 30 minutes. 40 $cm^3$ d'éther ont été ajoutés, et la couche organique a été lavée avec $H_2SO_4$ 1N et de la saumure; les couches aqueuses ont relavées avec de l'éther, séchées avec $Na_2SO_4$), rotoévaporées et distillées en vue de donner du VOC—$NMe_2$ pur, ayant son point d'ébullition à 57—61°C à 3,5 kPa (26 mm Hg). Il faut noter que Olofson, Schnur et Bunes (Brevet U.S. n° 3 905 981, ont rapporté: 43°C à 1,3 kPa (10 mm Hg).

IR (μ): 5,78 (vs), 6,08 (m); $CCl_4$

$^1H$ NMR (δ): 7,13 (d de d, J = 14, 6), 4,65 (d de d, J = 14, 1), 4,30 (d de d, J = 6, 1), 2,91 (s); rapport 1:1:1:6; $CCl_4$.

Dans deux expériences de contrôle RMN, la réaction a été effectuée respectivement pendant 30 minutes à 170°C, et pendant 1 heure à 140°C. Au cours de premier essai, 57% de produit ont été formés, mais le reste s'est décomposé. Dans le second essai, il est resté la plus grande partie du composé de départ, mais 26% de VOC—$NMe_2$ étaient présents, et une décomposition de 9% a été observée.

Exemple 25

Préparation de la N-vinyloxycarbonyl-N-méthyl-p-chloroaniline (N-VOC-N-méthyl-p-chloroaniline)

a) Préparation de la N-ACE-N-méthyl-p-chloroaniline

De la N-méthyl-p-chloroaniline (Aldrich, 94%) (19,9 g, 0,13 mole) et de al pyridine (10,5 g, 0,13 mole) ont été ajoutées à une solution refroidie (à 0°C), agitée de ACE—Cl (21,0 g, 0,15 mole) dans 25 $cm^3$ d'éther. Le mélange a été agité à la température ambiante pendant 1 heure, puis on l'a fait passer à travers un tampon de gel de silice en utilisant l'éther comme éluant. L'éluat a été évaporé, sous vide et l'huile jaune visqueuse restante a été placée sous vide à 133,3 Pa (1 mm) pendant une nuit pour enlever les matières volatiles. Le produit (32,3 g, rendement de 99%) était pur suivant l'analyse RMN.

IR (μ): 5,78 (vs), 6,25 (w), 6,68 (m); $CCl_4$

$^1H$ NMR (δ): 7,6—7,0 (m avec une grande pointe à 7,23), 6,52 (q, J = 6), 3,26 (s), 1,69 (d, J = 6); rapport 4:1:3:3; $CCl_4$

MS (m/e): 251,0104 (P[$^{37}Cl_2$], 4%, calc. 251,0108), 249,0142 (P[$^{37}Cl^{35}Cl$], 26%, calc. 249,0137), 247,0177 (P[$^{37}Cl_2$], 42%, calc. 247,0166), 185 (48%), 168 (53%), 140 (69%), 63 (100%).

b) Conversion de la N-ACE-N-méthyl-p-chloroaniline en N-VOC-N-méthyl-p-chloroaniline

Une solution de N-ACE-N-méthyl-p-chloroaniline (10,8 g, 0,44 mole), de (−)-β-pinène (9,4 g, 0,069 mole) et 27 $cm^3$ de o-dichlorobenzène a été chauffée (bain d'huile à 185°C) pendant 9 heures (suivant l'analyse RMN, l'élimination était complète à 22% après 3 heures), puis refroidie et distillée sous vide. La fraction de distillation, ayant pour point d'ébullition 75—95°C à 0,4 mm, contenait de la N-VOC-N-méthyl-p-chloraniline (rendement absolu 40%), contaminée par une grande quantité de N-méthyl-p-chloroaniline. L'amine a été éliminée par extraction avec du $H_2SO_4$ aqueux. La N-VOC-N-méthyl-p-chloro-aniline analytiquement pure avait un point d'ébullition de 93 à 95°C à 40 Pa (0,3 mm Hg).

IR (μ): 5,76 (vs), 6,05 (m), 6,67 (s); $CCl_4$

$^1H$ NMR (δ): 7,5—7,0 (m avec une grande pointe à 7,20), 4,61 (d ou d, J = 14, 1), 4,36 (d ou d, J = 6, 1), 3,22 (s); rapport 5:1:1:3; $CCl_4$

MS (m/e): 213,0732 (P[$^{37}Cl$], 9%, calc. 213,0370), 211,0402 (P[$^{35}Cl$], 30%, calc. 211,0400), 170 (31%), 168 (100%).

Exemple 26

Préparation de N-vinyloxycarbonyl-14-acétylnoroxycodone (N-VOC-14-acétyl-noroxycodone)

a) Préparation de N-ACE-14-acétyl-noroxycodone

Du ACE—Cl (4,6 g, 32 mmoles) dans 5 $cm^3$ de dichloroéthane-1,2 a été ajouté à une solution refroidie (à 0°C), agitée, de 14-acétyloxycodone (2,0 g, 5,7 mmoles), de bis-(diméthylamino)-1,8 naphtalène (0,2 g, 1

mmole) et de 15 $cm^3$ de dichloroéthane. Le mélange a été laissé à la température ambiante pendent 1 heure, puis agité pendant une nuit à 85°C. On a fait passer du HCl anhydre dans la solution refroidie pendant 2 minutes, puis le solvant et le ACE—Cl en excès ont été enlevés sous vide. Du charbon de bois et du dichlorométhane ont été ajoutés, et l'on a fait passer le mélange à travers un tampon de silice (en utilisant comme éluant $CH_2Cl_2$: MeOH 95: 5). L'évaporation sous vide du solvant a fourni due N-ACE-14-acétyl-noroxycodone, pur suivant RMN, sous forme de mousse jaunâtre, 2,1 g (rendement de 83%).

IR (μ): 5,65—5,9 (vs avec des maxima à 5,72 et 5,83); $CH_2Cl_2$

$^1H$ NMR (δ): 7,0—6,3 (m), 5,8—5,5 (large), 4,65 (s), 4,2—3,8 (m avec la pointe du méthyle à 3,90), 3,4—1,3 (m avec méthyle s à 2,17 et méthyle d à 1,84); rapport 3:1:1:4:15; $CDCl_3$

Le produit (N-ACE-acétyl-noroxycodone) à la structure suivante:

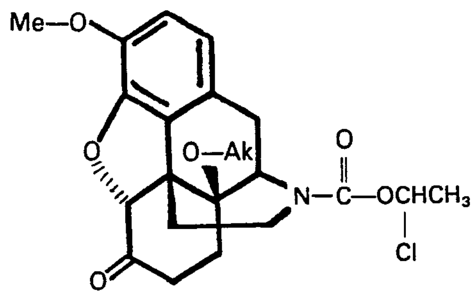

b) conversion du N-ACE-14-acétyl-noroxycodone en N-VOC-14-acétyl-noroxycordone

Une solution de N-ACE-14-acétyl-noroxycodone (2,1 g, 4,6 mmoles) et de collidine-2,4,6 (1,2 g, 10,1 mmoles) dans 4,8 $cm^3$ de bromobenzène a été chauffée (bain d'huile à 170°C) pendant 3 heures. Du méthanol (5 $cm^3$) a été ajouté à la solution rouge refroidie, que l'on a chauffée au reflux pendant 30 minutes. Le mélange a été dilué avec du chlorure de méthylène, lavé avec $H_2SO_4$ 1N et séché ($Na_2SO_4$). Du charbon de bois a été ajouté, et l'on a fait passer la bouillie à travers un tampon en gel de silice, en utilisant l'acétate d'éthyle comme éluant. L'évaporation sous vide a fourni le N-VOC-14-acétyl-noroxycodone sous forme d'un solide légèrement jaune, en quantité de 0,96 g (rendement de 51%). Après recristallisation dans du $CH_2Cl_2$-heptane et du méthanol, le produit présente un point de fusion de 180—182°C; il faut noter que Olofson et Pepe (Brevet U.S. 4 141 897) ont rapporté les valeurs: 181—182,5°C, 182,5—183,5°C. Les données spectrales IR et RMN obtenues pour le produit étaient en accord avec les valeurs indiquées par Olofson et Pepe, loc. cit.

Exemple 27

Préparation de la N-vinyloxycarbonyl-guvacoline (N-VOC-guvacoline)

a) Préparation de la N-ACE-guvacoline

On a versé goutte à goutte (pendant 10 minutes) de l'arécoline fraîchement distillée (2,08 g, 0,0134 mole), contenue dans 8 $cm^3$ de dichloroéthane, dans une solution agitée (à −5°C) de ACE—Cl (2,59 g, 0,0181 mole) et de bis-diméthylamino)-1,8-naphtalène (0,28 g, 0,00131 mole) dans 10 $cm^3$ de dichloroéthane. La solution a d'abord été chauffée jusqu'à la température ambiante, puis au reflux; pendant ce temps, un solide blanc a précipité. Après un reflux, de 30 minutes, le mélange devenu rougeâtre-orange a été refroidi; on a fait passer lentement du HCl anhydre (pendant 2 minutes) à travers le mélange, ce qui a provoqué la dissolution de solide; puis on a fait passer la solution à travers un tampon de gel de silice (2,5 cm × 2,5 cm), en utilisant du chlorure de méthylène comme éluant. La rotoévaporation de l'éluant total (125 $cm^3$) a fourni 3,20 g (rendement de 96%) de N-ACE-guvacoline brute sous forme d'huile dorée.

IR (μ): 5,81 (vs), 6,10 (w); $CH_2Cl_2$

NMR (δ): 7,3—6,8 (m), 6,58 (q, J = 6), 4,4—3,9 (m), 3,8—3,2 (m avec pointe de méthyle à 3,73), 2,6—2,1 (m), 1,83 (d, J = 6); rapport 1:1:2:5:2:3; $CDCl_3$.

b) Conversion de la N-ACE-guvacoline en N-VOC-guvacoline

Une solution de N-ACE-guvacoline (1,65 g, 6,66 moles), de collidine-2,4,6 (1,09 g, 8,99 mmoles) et de 3,2 $cm^3$ de bromobenzène a été chauffée (par un bain d'huile à 170°C) pendant 6,5 heures. Du méthanol a été ensuite ajouté à la solution refroidie, que l'on a chauffée au reflux ensuite pendant 30 minutes. De l'éther (20 $cm^3$) a été ajouté à la solution refroidie qui a été extraite avec $H_2SO_4$ 1N (3 × 15 $cm^3$) et 10 $cm^3$ de saumure. Les couches aqueuses ont été relavées avec de l'éther (2 × 10 $cm^3$), et ces phases organiques combinées ont été séchées (avec du $Na_2SO_4$), rotoévaporées, et distillées sous vide pour donner la N-VOC-guvacoline; on a obtenu 0,842 g de ce produit (rendement 60%), ayant son point d'ébullition à 120—122°C à 80 Pa (0,6 mm Hg). R.A. Olofson, R.C. Schnur et L.A. Bunes (Brevet U.S. 3 905 981) ont rapporté une valeur de 132°C à 106 Pa (0,8 mm Hg) et 104—106°C à 26,6 Pa (0,2 mm Hg).

IR (μ): 5,78 (vs), 6,02 (w), 6,06 (m); $CCl_4$

$^1H$ NMR (δ): 7,4—6,85 (m incluant d de d, J = 14, 7 à 7,15), 4,71 (d large, J = 14), 4,39 (d de d, J = 7, 1),

4,3—4,0 (m), 3,70 (s), 3,53 (t, J = 6), 2,6—2,1 (m): rapport 2:1:1:2:3:2:2; $CCl_4$.

La N-VOC-guvacoline a la formule:

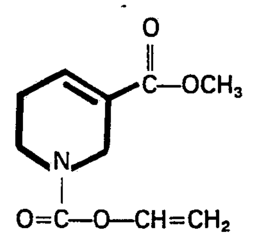

Exemple 28

Préparation de la N-vinyloxycarbonyl-O-acétyl-nortropine (N-VOC-O-acétyl-nortropine)

a) Préparation de la N-ACE-O-acétyl-nortropine

La N-déméthylation de la O-acétyl-tropine avec ACE—Cl, réalisée en utilisant le procédé décrit plus haut pour la déméthylation similaire de l'arécoline, a fourni la N-ACE-O-acétyl-nortropine sous forme d'huile jaune après évaporation sous vide de l'éluat chromatographique.

IR (μ): 5,81 (vs); $CH_2Cl_2$

NMR (δ): 6,60 (q, J = 6), 5,2—4,9 (m), 4,5—4,1 (m), 2,5—1,6 (m avec méthyle s à 2,03 et méthyle d à 1,80); rapport 1:1:2:14; $CDCl_3$.

b) Conversion de la N-ACE-O-acétyl-nortropine en N-VOC-O-acétyl-nortropine

Une solution de N-ACE-O-acétyl-nortropine (4,39 g, 0,016 moles) et de collidine-2,4,6 (2,36 g, 0,020 mole) dans 7,5 $cm^3$ de bromobenzène a été chauffée (bain d'huile à 170°C) pendant 3,5 heures. Dans une expérience antérieure utilisant l'analyse RMN, 73% du produit s'étaient formés an bout de 2 heures. La solution a été refroidie jusqu'à environ 50°C, diluée avec 10 $cm^3$ de méthanol et agitée à cette température pendant 45 minutes. La solution refroidie a été diluée avec 40 $cm^3$ d'éther, et lavée avec $H_2SO_4$ 1N (3 × 25 $cm^3$) et de la samure (15 $cm^3$). Les couches aqueuses ont été réextraites avec de l'éther (2 × 20 $cm^3$). Les phases organiques combinées ont été séchées (avec $Na_2SO_4$), rotoévaporées et distilliées sous vide. La N-VOC-O-acétyl-nortropine a été isolée sous forme d'huile incolore, en quantité de 3,26 g (rendement de 86%, produit pur selon RMN) avec un point d'ébullition de 125 à 127°C à 80 Pa (0,6 mm Hg).

IR (μ): 5,73 (vs), 5,80 (vs), 6,06 (m); $CCl_4$

$^1H$ NMR (δ): 7,17 (d de d, J = 14, 7), 5,2—4,8 (m), 4,65 (d de d, J = 14, 1), 4,5—4,1 (m avec d de d à 4,35), 2,4—1,5 (m avec méthyle s à 1,98); rapport 1:1:1:3:11; $CCl_4$.

La structure de la N-VOC-acétyl-nortropine est la suivante:

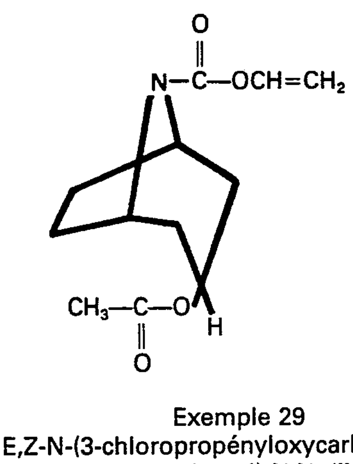

Exemple 29

Préparation de la E,Z-N-(3-chloropropényloxycarbonyl)-N,N-diisopropylamine

a) Préparation de la N-(1,3-dichloropropyloxycarbonyl)-N,N-diisopropylamine

De la diisopropylamine (9,4 g, 0,093 mole) dans 15 $cm^3$ d'éther a été ajoutée pendant 20 minutes à une solution refroidie (à 15°C) et agitée de 1,3-dichloropropyl-chloroformiate (point d'ébullition 65—68°C à 1,3 kPa (10 mm Hg) 7,1 g, 0,037 mole) dans 40 $cm^3$ d'éther. Après avoir agité pendant 30 minutes à la température ambiante, on a enlevé par filtration les sels précipités; le solvant a été évaporé, et le produit a été isolé par distillation sous vide; son point d'ébullition était de 88—92°C à 40 Pa (0,3 mm Hg); la quantité obtuenue était de 8,29 g (rendement de 87%). Le produit correspond à la formule: $ClCH_2CH_2CHCl—OC(=O)—N(CHMe_2)_2$.

IR (μ): 5,79 (vs); $CCl_4$

$^1H$ NMR (δ): 6,64 (t, J = 6), 4,3—3,4 (m, avec t de J = 7 à 3,65), 2,46 (q avec de larges pics au centre, J = 6, 7), 1,20 (d, J = 7); rapport 1:4:2:12; $CCl_4$

MS (m/e): 259 (1%), 257,0751 (P[$^{37}Cl$ $^{35}Cl$], 6%, calc. 257,0763), 255,0793 (P[$^{35}Cl_2$], 8%, calc. 255,0793),

244 (9%), 242 (54%), 240 (87%), 144 (15%), 130 (33%), 128 (45%), 43 (100%).

b) Préparation de la E,Z,N-(3-chloropropényloxycarbonyl)-N,N-diisopropylamine

Un ballon contenant une solution de $ClCH_2CH_2CHCl—OC(=O)—N(CHMe_2)_2$ (8,0 g, 0,031 mole), de collidine-2,4,6 (4,6 g, 0,038 mole) et de bromure de tétrabutylammonium (0,76 g, 0,002 mole) dans 17 $cm^3$ de tétrachloroéthylène a été soumise au reflux dans un bain d'huile maintenu à 125°C. (Suivant l'analyse RMN, la réaction était achevée à 41% 3 heures). La réaction a été poursuivie pendant 25 heures au total; le mélange a été dilué avec 40 $cm^3$ d'éther, puis extrait avec $H_2SO_4$ 1N (3 × 30 $cm^3$) et de la saumure (20 $cm^3$). Les couches aqueuses ont été réextraites avec de l'éther (2 × 20 $cm^3$). Les couches organiques combinées ont été séchées ($Na_2SO_4$), rotoévaporées et distillées sous vide. La fraction présentant un point d'ébullition à 98—99°C à 0,4 mm (en quantité de 4,67 g) contenait les isomères géométriques E et Z de la 3-(chloropropenyloxycarbonyl)-N,N-diisopropylamine obtenue avec un rendement de 56% et un rapport E: Z de 1: 1,7. D'après l'analyse RMN, le produit a également été contaminé par 16% mole de carbamate de dichloropropyle initial. En utilisant des durées de réaction plus longues, on a pu obtenir un produit exempt de ce dernier réactif. Les données spectrales pour le produit E,Z pur sont données ci-dessous. Le composé correspond à la la formule $ClCH_2CH=CHOC(=O)N(CHMe_2)_2$.

IR (μ): 5,81 (s), 5,99 (w); $CCl_4$

$^1H$ NMR (δ): 7,42 (d, isomère E, J = 12), 7,20 (d, isomère Z, J = 6), 5,7—4,7 (m), 4,3—3,4 (m), 1,33—1,18 (chevauchement entre d de J = 6 à 1,27 et d de J = 6 à 1,23); rapport 0,37:0,63:1:4:12; $CCl_4$

MS (m/e): 221 (P[$^{37}Cl$], 1%), 219,1031 (P[$^{35}Cl$], 3%, calc. 219,026), 128 (61%), 86 (81%), 43 (100%).

Cette expérience démontre que l'élimination due chlore dans la position 1 est possible (contrairement à ce que l'on attend), alors que l'atome de chlore se trouvant dans la position 3 n'est pas attaqué.

Exemple 30

Préparation du 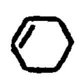

=CH-O-C(=O)-NH-n-butyle (mélange des isomères géométriques E,Z

a) Préparation du 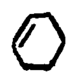

−CHCl-O-C(=O)-NH-n-butyle

De la n-butylamine (13,5 g, 0,18 mole) contenue dans 10 $cm^3$ d'éther a été ajoutée pendant une période de 30 minutes à une solution agitée, refroidie (à 0°C) de chloroformiate de (3-cyclohexényl)-chlorométhyle (fabriqué suivant le procédé général décrit dans la demande de Brevet européen n° 40153; point d'ébullition 81—83°C à 133,3 Pa (1 mm Hg) (16,6 g, 0,080 mole) dans 25 $cm^3$ d'éther. Le mélange a été agité à la température ambiante pendant une heure; on a fait passer du HCl anhydre à travers le mélange pendant 2 heures, les sels ont été séparés par filtration, et du charbon de bois a été ajouté au filtrat, qui a ensuite été évaporé sous pression réduite. On a fait passer le résidu à travers un tampon de gel de silice 5,1 cm × 5,1 cm (2″ × 2″) en utilisant l'acétate d'éthyle-dichlorométhane 1:1 comme éluant. L'évaporation sous vide a fourni 16,8 g (rendement de 86%) du produit dont la structure est indiquée dans le titre, sous forme d'une huile orange. Le produit n'a pas pu être distillé sous vide sans décomposition résultant du processsus d'élimination du HCl décrit ci-dessous.

IR (μ): 5,70 (vs); $CCl_4$

$^1H$ NMR (δ): 6,5—6,1 (m), 5,9—5,3 (m), 3,15 (large q, J = 6), 2,9—0,6 (m); rapport 1:3:2:14; $CCl_4$.

MS (m/e): 247,1143 (P[$^{37}Cl$], 1%, calc. 247,1153), 245,1181 (P[$^{35}Cl$], 3%, calc. 245,1182), 128 (24%), 118 (100%).

b) Préparation du 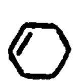

=CH-O-C(=O)-NH-n-butyle (mélange d'isoméres E,Z)

Un ballon contenant un échantillon du carbamate de chlororoalkyle de l'examplе 30 a/ (5,0 g, 0,02 mole) a été agité à 125°C sous un vide de 6,7 kPa (pas de réfrigérant à reflux) pendant quatre heures. Le liquide restant a été chromatographié sur une colonne de gel de silice 2,5 × 20,3 cm (1″ × 8″), en utilisant du dichlorométhane comme éluant. L'évaporation sous vide de l'éluat a donné 0,92 g (rendement de 21%, pur suivant RMN, CCM tache isolée de $R_f$ 0,48 développée avec du $CH_2Cl_2$ sur silice) du produit (paire d'isomères géométriques) dont la structure figure dans le titre, ceci sous forme d'une huile jaune.

IR (μ): 2,90 (m, élongation NH), 5,79 (vs, élongation C = O), 5,8—5,95 (absorptions m à w, élongation C = C); $CH_2Cl_2$

$^1H$ NMR (δ): 7,1—6,6 (large s), 5,8—5,4 (large s), 5,4—4,8 (large s), 3,5—0,6 (m); rapport 1:2:1:13; $CDCl_3$
MS (m/e): 209,1423 (P, 5%, calc. 209,1416), 128 (44%), 110 (100%), 86 (73%).

Le nom donné à la structure figurant dans le titre de l'exemple 30 b/ (et par conséquent du produit obtenu selon le procédé qui vient d'être décrit) est, d'après le "Chemical Abstract Service": "Carbamic acid, butyl, 3-cyclohexen-1-ylidene-methyl ester" (N-butyl carbamate de cyclohexène-3-ylidène-1-méthyle).

Exemple 31
Préparation de la E,Z,N-(3-méthyl-1-(buténylоxycarbonyl)-N-(4-chlorobutyl)-N-éthylamine

a) Préparation de la N-(1-chloro-3-méthyl-butyloxycarbonyl)-N-(4-chlorobutyl)-N-éthylamine

Une solution de N-éthylpyrrolidine (7,1 g, 0,071 mole) dans 15 $cm^3$ de dichloroéthane-1,2 a été a joutée pendant 15 minutes à une solution agitée, refroidie (à 0°C), de chloroformiate de chloro-1-méthyl-3-butyle (préparé à partir de l'isovaléraldéhyde par le procédé général décrit dans la demande de Brevet européen n° 40153; point d'ébullition 74—76°C à 5,3 kPa (30 mm Hg) (11,1 g, 0,06 mole) dans 25 $cm^3$ de dichloroéthane, contenant également du bis-(diméthylamino)-1,8-naphtalène (0,94 g, 0,004 mole). On a chauffé au reflux le mélange pendant 30 minutes, puis on l'a fait refroidir, et l'on a fait passer du HCl anhydre, lentement, à travers la solution pendant 2 minutes. La rotoévaporation du solvant a fourni un résidu à partir duquel le produit a été isolé et purifié par chromatographie à travers une colonne de gel de silice 15,2 cm × 2,5 cm (6" × 1") en utilisant l'acétate d'éthyle comme éluant. L'évaporation sous vide de l'éluat a donné une huile jaune; une quantité de 16,4 g (rendement de 96%) a été identifiée comme étant le produit $(CH_3)_2\,CHCH_2—CHCl—OC(=O)—N(CH_2CH_3)—CH_2CH_2CH_2CH_2Cl$; point d'ébullition: 119—122°C à 53 Pa (0,4 mm Hg).

IR (μ): 5,82 (vs); $CH_2Cl_2$

$^1H$ NMR (δ): 6,47 (t, J = 6), 3,7—2,9 (m), 2,1—1,3 (m), 1,20—0,82 (chevauchement entre t de J = 7 à 1,05 et d de J = 6 à 0,87); rapport 1:6:7:9; $CDCl_3$

MS (m/e): 287,1052 (P[$^{37}Cl_2$], 0,2%, calc. 287,1047), 285,1106 (P[$^{37}Cl^{35}Cl$], 1%, calc. 285,1077), 283,1119 (P[$^{35}Cl_2$], 2%, calc. 283,1106), 206 (51%), 102 (51%), 69 (100%).

b) Préparation de la E, Z, N-(3-méthyl-1-buténylоxycarbonyl)-N-(4-chlorobutyl)-N-éthylamine

on a chauffé au reflux pendant 12 heures un mélange de N-(1-chloro-3-méthyl-butyloxycarbonyl)-N-(4-chlorobutyl)-N-éthylamine (6,32 g, 0,022 mole), de collidine-2,4,6 (3,32 g, 0,027 mole) et de bromure de tétrabutylammonium (0,55 g, 0,002 mole), dans 10 $cm^3$ de tétrachloroéthylène. (Suivant l'analyse RMN, la réaction était achevée à 43% au bout d'une heure). Le mélange refroidi a ensuite été versé sur une colonne de chromatographie en gel de silice 15,2 cm × 2,5 cm (1" × 6"), et élué avec de l'acétate d'éthyle. L'évaporation sous vide de l'éluat a fourni produit purifié sous forme d'huile jaune, en quantité de 5,20 g (rendement de 94%). La distillation sous vide du produit (point d'ébullition 111—114°C à 53 Pa (0,4 mm Hg)) a fait partir la couleur, mais par ailleurs n'a pas modifié la pureté. Le rapport entre les isomères cis et trans, dans le produit $(CH_3)_2CHCH=CH—OC(=O)—N(CH_2CH_3)—CH_2CH_2CH_2CH_2Cl$, a été déterminé par analyse RMN (z : E = 4 : 3); il est resté le même avant et après l'étape de distillation.

IR (μ): 5,81 (vs), 5,95 (m); $CCl_4$

$^1H$ NMR (δ): 7,2—6,7 (m), 5,25 (d de d, J = 12, 7, isomère E), 4,60 (d de d, J = 9, 6, isomère Z), 3,6—3,0 (m), 2,7—1,3 (m), 1,30—0,97 (chevauchement entre t de J = 7 à 1,15 et d de J = 7 à 1,00); rapport 1:0,43:0,57:6:5:9; $CDCl_3$

MS (m/e): 249,1337 (P[$^{37}Cl$], 2%, calc. 249,1310), 247,1338 (P[$^{35}Cl$], 5%, calc. 247,1339), 164 (9%), 162 (29%), 93 (32%), 91 (100%).

Cette expérience montre que l'élimination de HCl a partir d'une N-(1-chloroalcoxycarbonyl)-amine, pour obtenir la N-(1-alcényloxycarbonyl)-amine, est possible, contrairement à ce que l'on attend, alors qu'un autre atome de chlore, attaché à un autre substituant chloroalkyle (ici un groupe 4-chlorobutyle) sur le même azote n'est pas attaqué.

Exemple 32
Préparation de la N,N'-di-(3-méthyl-buténylоxycarbonyl)-N-N'-diméthyl-1,3-propane-diamine

a) Préparation de la N,N'-di-(1-chloro-3-méthyl-butyloxycarbonyl)-N,N'-diméthyl-1,3-propane-diamine

La réaction de la N,N,N',N'-tétraméthyl-propanediamine (6,86 g, 0,053 mole) avec le chloroformiate de chloro-1-méthyl-3-butyle (17,7 g, 0,096 mole) dans de dichloroéthane (quantité totale de 40 $cm^3$) en présence du bis(diméthyl-amino)-1,8 naphtalène (1,2 g, 0,006 mole), suivant la description donnée dans l'exemple 31 a/, suivie du traitement du mélange réactionnel et de la purification chromotographique du produit (également suivant la description donnée dans l'exemple 31 a/) a fourni, après évaporation sous vide de l'éluat, 15,6 g (rendement de 82%, pur suivant RMN) du produit.

IR (μ): 5,82 (vs); $CH_2Cl_2$

$^1H$ NMR (δ): 6,47 (t large, J = 6), 3,29 (large t, J = 7), 2,92 (s), 2,2—1,5 (m), 0,93 (d, J = 6); rapport 2:4:6:8:12; $CDCl_3$.

MS (m/e): 400, 1744 (P[$^{37}Cl^{35}Cl$], 0,5%, calc. 400,1709) 398,1762 (P[$^{35}Cl_2$], 1%, calc. 398,1739), 277 (8%), 154 (14%), 128 (100%).

b) Préparation de la N'N'-di-(3-méthyl-buténylоxycarbonyl)-N,N'-diméthyl-1,3-propanediamine

Une solution de N,N'-di-(1-chloro-3-méthyl-butyloxycarbonyl)-N,N'-diméthyl-1,3-propane-diamine (6,19 g, 0,016 mole), de collidine-2,4,6 (4,4 g, 0,036 mole) et de bromure de tétrabutylammonium (0,65 g, 0,002 mole) dans 13 $cm^3$ de tétrachloroéthylène a été soumise au reflux pendant 12 heures. (Une réaction a plus petite échelle était achevée à 44% au bout d'une heure, suivant l'analyse RMN). Le mélange a été refroidi, versé sur une colonne de gel de silice 15,2 cm × 2,5 cm (6" × 1") et chromatographié avec l'acétate d'éthyle comme éluant. L'évaporation sous vide de l'éluat a donné une huile foncée, en quantité de 4,70 g (rendement de 93%, produit pur suivant RMN); celle-ci a été identifiée comme étant le produit $(CH_3)_2CHCH=CH—OC(=O)—N(CH_3)—(CH_2)_3—(CH_3)N—C(=O)O—CH=CHCH(CH_3)_2$. Sur la base de l'analyse RMN, les géométries aux liaisons doubles carbone-carbone étaient 43% trans et 57% cis. Donc, le mélange calculé d'isomères géométriques est: 18% EE, 40% EZ et 32% ZZ, en admettant qu'il n'y a pas d'interactions sur longue distance. Si l'on se fonde sur le rapport d'isomères trouvé dans l'exemple 31 b/, une telle interaction sur longue distance serait extrêmement improbable. Une légère décomposition du produit (suivant l'analyse RMN) s'est produite au cours de la distillation sous vide; point d'ébullition 163—171°C à 53 Pa (0,4 mm Hg).

IR (μ): 5,81 (vs), 5,96 (m), $CCl_4$

$^1H$ NMR (δ): 7,2—6,7 (m), 5,25 (d de d, J = 12, 7, géométrie E), 4,58 (d de d, J = 9, 6, géométrie Z), 3,32 (large t, J = 7), 2,96 et 2,93 (singulets se chevauchant pour différentes conformations N—Me), 2,7—1,5 (m), 1,00 (d, J = 6); rapport 2:0,86:1,14:4:6:4:12; $CDCl_3$

MS (m/e): 326,2210 (P, 3%, calc. 326,2206), 241 (35%), 184 (44%), 129 (100%).

Exemple 33

Préparation du N,N'-di-(3-méthyl-buténloxycarbonyl)-1,10-diaza-18-couronne-6

a) Préparation du N,N'-di-(1-chloro-3-méthyl-butyloxycarbonyl)-1,10-diaza-18-couronne-6

Une solution de chloroformiate de chloro-1 méthyl-3-butyle (1,58 g, 8,54 mmoles) dans le dichlorométhane (10 $cm^3$) a été ajoutée à une solution refroidie (à 0°C) et agitée de 1,10-diaza-18-couronne-6 (0,99 g, 3,75 mmoles) et de pyridine (0,68 g, 8,60 mmoles) dans le dichlorométhane (10 $cm^3$). Le mélange de réaction a été chauffé jusqu'à la température ambiante, agité pendant une heure et versé su un tampon de gel de silice (élué avec de l'acétate d'éthyle). Après évaporation sous vide de l'éluat, et après avior séché le résidu sous vide pendant une nuit, il subsistait une huile limpide; la quantité obtenue était de 1,78 g (rendement de 85%, produit pur suivant RMN $H^1$.

IR (μ): 5,82 (vs); $CH_2Cl_2$

$^1H$ NMR (δ): 6,50 (large t, J = 6), 3,60 (large s), 2,2—1,6 (m), 0,95 (large d, J = 6); rapport 2:24:6:12; $CDCl_3$.

MS (m/e): 562 ($P[^{37}Cl_2]$, 0,2%), 560,2402 ($P[^{37}Cl^{35}Cl]$, 0,8%, calc. 560,2444), 558,2452 ($P[^{35}Cl_2]$, 1.2%, calc. 558,2475), 351 (19%), 289 (11%), 158 (25%), 114 (100%).

b) Préparation de N,N'-di-(3-méthyl-buténylоxycarbonyl)-1,10-diaza-18-couronne-6

On a chauffé au reflux un mélange de N,N'-di-(1-chloro-3-méthyl-butyloxycarbonyl)-1,10-diaza-18-couronne-6 (0,64 g, 1,14 mmoles), de collidine (0,40 g, 3,30 mmoles) et de bromure de tétrabutylammonium 0,055 g, 0,17 mmoles) dans le tétrachloroéthylène (1,3 $cm^3$) pendant 8,5 heures. Le mélange réactionnel refroidi a été versé sur un tampon de gel de silice et élué avec de l'acétate d'éthyle. On a obtenu 0,40 g d'une huile jaune, après avoir séché le résidu sous vide à 80°C pendant une nuit (rendement de 71%, produit pur suivant RMN 1H avec un rapport E: Z de 0,68: 1).

IR (μ): 5,81 (vs), 5,98 (m); $CCl_4$

$^1H$ NMR (δ): 7,2—6,8 (m), 5,26 (d de d, J = 12, 7, géométrie E), 4,63 (d de d, J = 9, 6, géométrie Z), 3,60 (large s), 2,8—2,0 (m), 1,02 (d, J = 6); rapport 2:0,81:1,19:24:2:12; $CDCl_3$

MS (m/e): 486,2907 (P, 3%, calc. 486,2941), 401 (48%), 357 (95%), 114 (100%).

La structure du produit obtenu dans b) est:

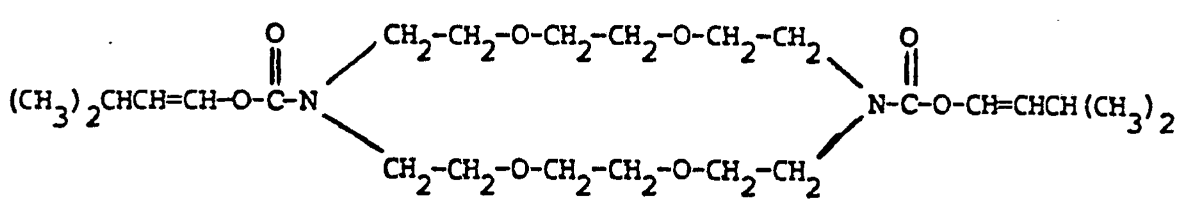

De façon similaire on fait réagir le 1,10-diaza-18-couronne-6 avec le chloroformiate d'α-chloroéthyle pour obtenir le composé intermédiaire N,N'-di-α-chloroéthyle qui est déshydrohalogéné comme à l'exemple 21 b/ pour récupérer le N,N'-di-(vinyloxycarbonyl)-1,10-diaza-18-couronne-6.

Exemple 34

Préparation de la N-(Isobuténylоxycarbonyl)-N'-méthyl-pipérazine

a) Préparation de la N-(α-chloroisobutyloxycarbonyl)-N'-méthyl-pipérazine

Du chloroformiate d'α-chloroisobutyle (9,51 g, 0,56 mole) dans 15cm$^3$ de dichloroéthane a été ajouté (pendant 20 minutes) à une solution refroidie (à −5°C) et agitée de N,N'-diméthyl-pipérazine (11,7 g, 0,102 mole) dans 25 cm$^3$ de dichloroéthane. La mélange de réaction a été agité à la température ambiante (pendant 1 heure), puis on l'a chauffé au reflux (pendant 30 minutes). Après élimination du solvant, le produit a été purifié par passage à travers un tampon de gel de silice (en utilisant l'acétate d'éthyle comme éluant). Après évaporation de l'éluat, on obtient une huile jaune (elle s'est par la suite solidifiée au repos); la quantité obtenue était de 11,2 g (rendement de 86%, produit pur selon RMN H$^1$).

IR (μ): 3,56 (w), 5,78 (vs); $CCl_4$

$^1$H NMR (δ): 6,31 (d, J = 5), 3,51 (t, J = 5), 2,6—1,8 (m avec t de J = 5 à 2,36 et méthyle s à 2,28), 1,05 (d, J = 6); rapport 1:4:8:6; $CDCl_3$

MS (m/e): 236,1113 (P[$^{37}$Cl], 6%, calc. 236,1106), 234,1140 (P[$^{35}$Cl], 20%, calc. 234,1135), 127 (73%), 70 (100%).

b) Préparation de la N-(isobutyloxycarbonyl)-N'-méthyl-pipérazine

Un mélange de *N-(α-chloroisobutyloxycarbonyl)-N'-méthyl-pipérazine* (10,6 g, 0,0452 mole) et de bromure de tétrabutyl-ammonium (0,90 g, 0,003 mole), a été chauffé sans solvant pendant 3 heures à 125°C et 113,3 Pa (1 mm Hg). Le mélange a été chauffé au reflux pendant env. 15 minutes, puis s'est solidifié. De l'eau (15 ml) et 10 cm$^3$ de dichlorométhane ont été ajoutés au récipient de réaction refroidi. Du $K_2CO^3$ solide en excès a ensuite été ajouté lentement au mélange agité (pour obtenir un pH ⩾ 11). La phase organique a été séparée et lavée avec 10% de solution de $K_2CO_3$ (50 cm$^3$). Les extraits aqueux combinés ont été lavés avec du dichlorométhane (2 × 20 cm$^3$) et les couches de dichlorométhane ont été combinées, séchées (avec du $Na_2SO_4$), rotoévaporées et distillées sous vide; la quantité obtenue était de 5,67 g (rendement de 63%, produit pur suivant RMN H$^1$), avec un point d'ébullition de 94 à 99°C à 0,7 mm.

IR (μ): 3,55 (w), 5,81 (vs); $CCl_4$

$^1$H NMR (δ): 6,9—6,6 (m), 3,53 (t, J = 5), 2,45—2,48 (chevauchement entre t de J = 5 à 2,34 et méthyle s à 2,28, 1,63 (large s); rapport 1/4:7:6; $CDCl_3$

MS (m/e): 198,1364 (p, 26%, calc. 198,1368), 127 (100%), 98 (9%), 70 (13%).

Dans une autre réaction à plus petite échelle, effectuée de la même manière, le produit a été purifié en faisant passer le résidu (après traitement au $K_2CO_4$ aqueux et évaporation du solvant) à travers un tampon de gel de silice (acétate d'éthyle comme éluant). Une huile jaune, contenant un peu d'un solide blanc, a été isolé après rotoévaporation de l'éluat (85% de rendement brut). Le solide a été trituré avec de l'hexane, et un produit solide blanc a été obtenu (point de fusion 149—151°C). L'analyse spectrale a montré que le sous-produit obtenu était de la N,N'-di-(isobuténylοxycarbonyl)-pipérazine.

IR (μ): 5,85 (vs); $CH_2Cl_2$

$^1$H NMR (δ): 6,78 (large s), 3,53 (s), 1,63 (large s); rapport 2:8:12; $CDCl_3$

MS (m/e): 282,1567 (P, 18%, calc. 282,1580), 211 (100%), 139 (26%), 55 (65%).

De manière similaire, on fait réagir la N,N'-diméthylpipérazine avec le chloroformiate d'α-chloro-éthyle (ACE—Cl) pour obtenir le composé intermédiaire N-α-chloro-éthyle qui est ensuite déshydrohalogéné comme à l'exemple 21 b) pour récupérer la N-(vinyloxycarbonyl)-N'-méthyl-pipérazine.

Exemple 35

Préparation du chlorure de N-3-[N'-(isobuténylοxycarbonyl)-N'-méthylamino]-propyl-triméthylammonium

a) Préparation du chlorure de (3-diméthylaminopropyl) triméthylammonium

De l'iodure de (3-diméthylaminopropyl)-triméthylammonium a été fabriqué suivant le procédé décrit par Seeman et Bassfield dans J. Org. Chem. *42,* 2337 (1977); point de fusion 171,5—172,5°C (point de fusion tit. 173,5—174,5°C). Une quantité de 3,76 g (13,8 mmoles) a été dissoute dans 95% d'éthanol, et on l'a fait passer à travers une colonne de résine échangeuse d'ions ("Amberlite IRA—400 C.P.", $RN^+ME_3Cl^-$, 35 g, 150,5 mmoles) avec 95% d'éthanol comme éluant. L'eluat a été rotoévaporé et le produit obtenu (chlorure du titre) a été séché sous vide à 80°C; on a obtenu 2,48 g (rendement de 99%) d'une poudre blanche (point de fusion 146—148°C).

$^1$H NMR (μ): 3,9—3,4 (m avec pointe à 3,47), 2,6—1,7 (m avec pointe à 2,20); rapport 11:10; $CDCl_3$.

b) Préparation du chlorure de N-3- [N'(α-chloroisobutyloxy-N'-méthylamino]-propyl-triméthylammonium

Une solution de chloroformiate d'α-chloroisobutyle (2,40 g, 14,0 mmoles) dans 10 cm$^3$ de dichloroéthane a été ajoutée (pendant 10 minutes) à un mélange refroidi (à 0°C) et agité de chlorure de 3-diméthylaminopropyl-triméthylammonium (2,48 g, 13,7 mmoles) dans 10 cm$^3$ de dichloroéthane. Le mélange réactionnel a été chauffé à 75°C pendant 30 minutes, refroidi et filtré. Le filtrat a été concentré, et il est resté une huile légèrement brune (3,94 g). L'analyse (RMN H$^1$) a montré que le mélange obtenu contenait le produit chloré du titre de b), ainsi qu'un peu de carbamate d'isobuténylе (respectivement 2:1). Le rendement combiné, pour les deux carbamates était de 95%. Les spectres, pour lel carbamate de chloroisobutyle pur, sont données ci-dessous.

IR (μ): 5,82 (vs); $CH_2Cl_2$

$^1$H NMR (δ): 6,3—6,1 (m), 3,9—2,8 (m avec pointe $N'^+Me_3$ à 3,47 et pointe N—Me à 3,02), 2,5—1,8 (m), 1,09 (large d, J = 6); rapport 1:16:3:6; $CDCl_3$

c) Préparation du chlorure de N-3-[N'-(isobuténytoxycarbonyl)-N'-méthylamino]-propyltriméthylammonium

Le mélange obtenu ci-dessus (3,81 g, 13,3 mmoles) a été chauffé au reflux dans 20 $cm^3$ de dichloroéthane. Un morceau de papier pH maintenu an-dessus du tube de séchage au $CaCl_2$ a permis de constater que du gaz HCl se dégageait du mélange chauffé. Au bout de 4,5 heures, le mélange réactionnel a été refroidi et filtré. La gomme obtenu, après concentration du filtrat, a été identifiée comme étant le produit du titre (RMN $H^1$ pur, 3,42 g, rendement de 97%), dont la structure est:

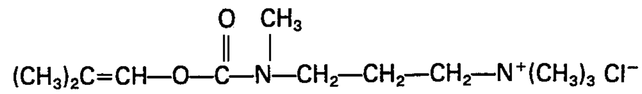

IR (µ): 5,84 (vs); $CH_2Cl_2$

$^1H$ NMR (δ): 6,65 (large s), 4,2—2,8 (m avec pointe $N'^+Me_3$ à 3,45 et pointe N—Me à 3,01), 2,6—1,9 (m), 1,65 (large s); rapport 1:16:2:6; $CDCl_3$

MS (m/e): 214,1680 (P—MeCl, 1,2%, calc. 214,1681), 143 (23%), 84 (38%), 58 (100%)

Dans une autre expérience, la carbamate de chloroisobutyle intermédiaire n'a pas été isolé. Après la déméthylation du chlorure de 3-diméthylaminopropyl-triméthylammonium (4,17 g, 23,1 mmoles) avec du chloroformiate de chloroisobutyle (5,50 g, 32,2 moles) dans le dicholoroéthane, suivie de la filtration et la concentration du filtrat (comme plus haut), le résidu a été chauffé à 70° à env. 133,3 Pa (1 mm Hg) pendant 2 jours. L'analyse (RMN $H^1$) du mélange obtenu a indiqué un rapport de 4,4:1 entre l'isobutényle et les carbamates de chloroisobutyle (rendement corrigé de 75%).

De maniére similaire on fait réagir le chlorure de 3-diméthylaminopropyle (triméthylammonium avec le chloroformiate d'α-chloroéthyle (ACE—Cl) pour obtenir l'intermédiaire α-chloroéthyle qui est ensuite déshydroholgéné comme à l'exemple 21 b) pour récupérer le chlorure de N-3-[N'-(vinyloxycarbonyl)-N'-methylamino]-propyltriméthyl ammonium.

## Exemple 36

### Préparation du carbonate de N,N'-di-VOC-N,N'-di-(2-méthylaminoéthyle)

a) Préparation du carbonate de N,N'-di-ACE-N,N'-di-(2-méthylaminoéthyle

Une solution de ACE—Cl (12,6 g, 0,088 mole) dans 15 $cm^3$ de dichloroéthane a été ajoutée pendant 15 minutes à une solution refroidie (0°C) et agitée de carbonate de 2-diméthylamino-éthyle (préparée suivant la description donnée par Angier et al., J. Med. Chem. *11,* 720 (1968)) (8,65 g, 0,042 mole) et d' "Eponge à proton" (0,35 g, 0,002 mole) dans le dichloroéthane (25 $cm^3$). Le mélange de réaction a été agité à la température ambiante pendant 1 heure, puis on l'a chauffé au reflux pendant 30 minutes. On a fait passer du HCl anhydre dans le mélange refroidi pendant 2 minutes, puis le solvant et le ACE—Cl en excès ont été éliminés sous vide. Une huile légèrement jaune a été obtenue après que l'on ait fait passer le résidu à travers un tampon en gel de silice (l'acétate d'éthyle servant d'éluant) et que l'éluat ait été évaporé sous vide; la quantité obtenue était de 9,70 g (rendement de 59%, pur suivant RMN $H^1$).

IR (µ): 5,70 (s), 5,78 (vs); $CCl_4$

$^1H$ NMR (δ): 6.43 (q, J = 6), 4,19 (large t, J = 5), 3.49 (large t, J = 5), 2,95 (s), 1,77 (d, J = 6); rapport 2:4:4:6:6; $CDCl_3$

b) Préparation du carbonate de N,N'-di-VOC-N,N'-di-(2-méthylaminoéthyle)

On a chauffé au reflux pendant 75 minutes un mélange de carbonate de N-N'-di-ACE-N,N'-di-(2-méthylaminoéthyle) (8,51 g, 0,022 mole) et de collidine (5,91 g, 0,049 mole) dans 15 $cm^3$ d'o-dichlorobenzène. Le solvant et la collidine en excès ont été éliminés sous vide, et l'on a fait passer le résidu rouge foncé à travers un tampon de gel de silice (en utilisant l'acétate d'éthyle comme éluant. L'évaporation de l'èluat, suivie de la distillation sous vide de l'huile restante a fourni une fraction, ayant son point d'ébullition à env. 170—180°C à 8 Pa (0,6 mm Hg) (1,59 g, rendement de 23%), qui a été identifiée comme étant le produit di-VOC du titre.

IR (µ): 5,70 (s), 5,78 (vs), 6,06 (m); $CCl_4$

$^1H$ NMR (δ): 7,00 (d de d, J = 14, 6), 4,9—3,8 (m avec t de J = 5 à 4,14), 3,46 (large t, J = 5), 2,95 (large s); rapport 2:8:4:6; $CCl_4$

MS (m/e): 316 (P, 0,1%), 273,1081 (P—OCH=$CH_2$, 6%, calc. 273,1087), 172 (4%), 128 (100%), 102 (60%)

## Exemple 37

### Préparation du N,N'-di-(isobuténytoxycarbonyl)-dibenzo-1,4-dioxa-8,12-diaza-cyclopentadéca-5,4-diène

a) Préparation du N,N'-di-(α-chloroisobutyloxycarbonyl)-dibenzo-1,4-dioxa-8,12-diaza-cyclopentadéca-5,14-diène

Une solution de chloroformiate d'α-chloroisobutyle (0,68 g, 3,98 mmoles) dans 7 $cm^3$ de dichlorométhane a été ajoutée pendant 10 minutes à une solution refroidie (à 0°C), agitée, de dibenzo-1,4-dioxa-8,12-diaza-cyclopentadéca-5,14-diène (provenant de la société Fluka, 0,48 g, 1,54 mmoles) et de pyridine (0,26 g, 3,29 mmoles) dans 7 $cm^3$ de dichlorométhane. Après avoir agité pendant une nuit à la

température ambiante, on a versé le mélange réactionnel jaune sur un tampon de gel de silice, et on l'a élué avec de l'acétate d'éthyle. L'éluat a été rotoévaporé et le résidu a été séché sous vide, fournissant le produit sous forme de poudre blanche, en quantité de 0,84 g (rendement de 94%); point de liquéfaction 54—58°C.

IR (μ): 5,85 (vs), 6,29 (w), 7,07 (vs); $CHCl_2$

$^1H$ NMR (δ): 7,6—6,7 (m), 6,5—6,2 (m), 4,56 (s), 4,40 (s), 3,5—2,9 (m), 2,5—0,7 (m avec d de J = 6 à 1,05); rapport 8:2:4:4:4:16; $CDCl_3$.

b) Préparation du N,N'-di-(isobuténylоxycarbonyl)-dibenzo-1,4-dioxa-8,12-diaza-cyclopentadéca-5,14-diène

On a chauffé au reflux pendant 4 heures un mélange du produit obtenu dans l'exemple 35 a) (0,75 g, 1,29 mmoles), de bromure de tétrabutylammonium (0,057 g, 0,18 mmoles) et de collidine (0,52 g, 4,29 mmoles) dans 1,1 $cm^3$ de tétrachloroéthylène. Le solvant et la collidine en excès ont été éliminés sous vide du mélange réactionnel à 100°C, et le résidu a été dilué avec du dichlorométhane et passé à travers un tampon en gel de silice, en utilisant l'acétate d'éthyle comme éluant. Une rotoévaporation de l'éluat, suivie d'un séchage sous vide, a fourni le produit du titre sous forme de gomme légèrement jaune (0.64 g, rendement de 98%).

IR (μ): 5,88 (vs), 6,92 (s); $CH_2Cl_2$

$^1H$ NMR (δ): 7,5—6,5 (m), 4,51 (s), 4,32 (s), 3,4—2,9 (m), 2,5—1,4 (m avec large s à 1,58); rapport 10:4:4:4:14; $CDCl_3$

Le produit final de l'exemple 37 b) a la struture suivante:

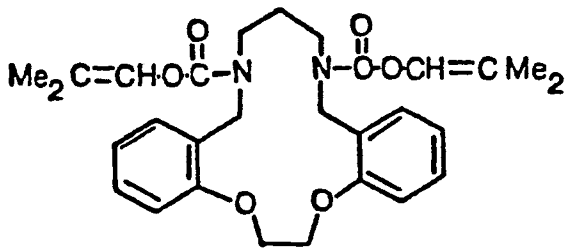

D'une façon similaire on a fait réagir le dibenzo-1,4-dioxa-8,12-diazacyclopentadéca-5,14-diène avec le chloroformiate d'α-chloroéthyle et on a obtenu le composé intermédiaire N,N'-di-α-chloroéthyl que l'on a déshydrohalogéné comme à l'exemple 21 pour récupérer le di-vinyloxycarbonyl dibenzo-1,4-dioxa-8,12-diaza-cyclopentadéca-5,14 diène.

**Revendications**

1. Procédé de préparation des carbamates vinyliques de formule I:

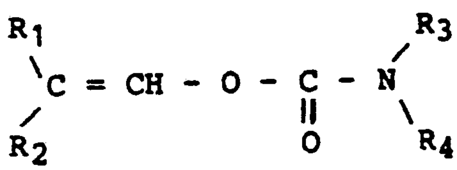

(I)

dans laquelle $R_1$ et $R_2$, identiques on différents représentent

un atome d'hydrogène,

un radical aliphatique saturé ou non, substitué ou non, ou bien

$R_1$ et $R_2$, conjointement avec l'atome de carbone auquel ils sont attachés, forment un cycle qui est saturé ou non, substitué ou non,

$R_3$ et $R_4$ sont identiques ou différents et représentent

un atome d'hydrogène,

un radical aliphatique, cycloaliphatique, substitué ou non, saturé on non, l'insaturation se trouvant sur un atome de carbone qui n'est pas adjacent à l'atome d'azote,

un radical de formule

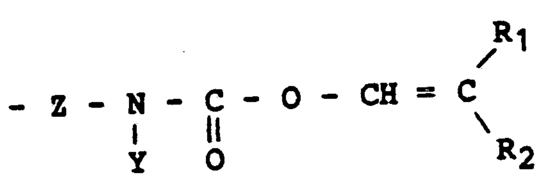

dans laquelle Y est un radical alkyle, $R^1$ et $R^2$ sont définis comme ci-dessus et Z est une chaîne hydrocarbonée comportant 2 à 20 atomes de carbone ou Z est une chaîne hydrocarbonée constituée de 2 à

20 atomes de carbone et contenant en outre des hétéroatomes W, W étant 0,S,NR'', chaque hétéroatome étant séparé d'un autre hétéroatome par au moins deux atomes de carbone et R'' e'tant un radical hydrocarboné ou Z est un groupe

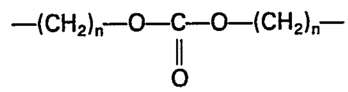

et n est un nombre entier compris entre 2 et 10,
un radical aromatique substitué on non ou bien,

$R^3$ et $R^4$ forment conjointement avec l'atome d'azote auquel ils sont attachés, un hétérocycle saturé ou non, substitué ou non,
$R^3$ et $R^4$ forment conjointement avec l'atome d'azote auquel ils sont attachés, un hétérocycle qui fait partie d'un système cyclique condensé saturé ou non, substitué ou non,
$R^3$ et $R^4$ forment conjointement avec l'atome d'azote auquel ils sont attachés, un hétérocycle qui contient au moins un autre hétéroatome qui est O, S, N ou $NR_x$, $R_x$ étant un radical hydrocarboné, et/ou un atome d'azote qui forme un groupe carbamate vinylique,
$R^3$ et $R^4$ forment conjointement avec l'atome d'azote auquel ils sont attachés un cycle de formule

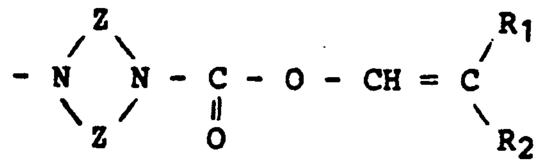

dans laquelle Z, $R_1$ et $R_2$ ont la signification précédente,
$R_3$ et $R_4$, forment conjointement avec l'atome d'azote auqel ils sont attachés un cycle pipérazinique, les deux atomes d'azote du cycle pipérazinique portant le groupe de formule:

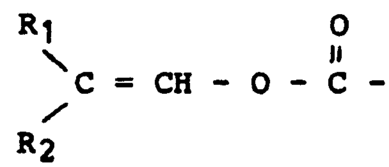

dans laquelle $R_1$ et $R_2$ ont la signification précédente,
qui consiste à chauffer un carbamate alpha-halogéné de formule (II):

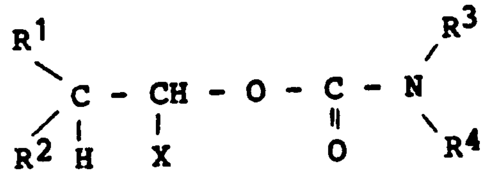
(II)

dans laquelle $R^1$, $R^2$, $R^3$ et $R^4$ sont définis comme précédemment et grand dans le composé de formule (I), $R^3$ et $R^4$ représentent un radical qui contient un groupe carbamate vinylique, ce dernier est présent dans le composé de formule (II) sous la forme d'un carbamate saturé contenant un atome d'hydrogène en position bêta et X en position alpha, X est un atome d'halogène, à une température comprise entre 80°C et 200°C pendant une période comprise entre quelques minutes et plusieurs heures.

2. Procédé suivant la revendication 1 où X est du chlore ou du brome.

3. Procédé selon la revendication 1, caractérisé en ce qu'on effectue la réaction en présence d'un catalyseur qui est un sel facilement ionisable, dont l'anion est non nucléophile ou faiblement nucléophile.

4. Procédé selon la revendication 3, caractérisé en ce que le cation dudit sel est choisi dans le groupe constitué de: a) un cation métallique; b) un cation métallique complexé avec un éther couronne; c) un cation métallique complexé avec cryptant; d) un caiton d'onium non substitué; e) un cation d'onium substitué par au moins un radical organique ayant de 1 à 7 atomes de carbone.

5. Procédé selon la revendication 4, caractérisé en ce que le cation est un cation de métal alcalin ou alcalino-terreux.

6. Procédé selon la revendication 4, caractérisé en ce que le cation onium est un cation ammonium, phosphonium, arsonium ou sulfonium.

7. Procédé selon la revendication 4, caractérisé en ce que le cation onium est un cation de tétra-n-butylammonium.

8. Procédé selon la revendication 3, caractérisé en ce que l'anion est choisi dans le groupe constitué par

les halogénures, $ClO_4^-$, et $NO_3^-$.

9. Procédé selon la revendication 8, caractérisé en ce que l'halogénure est un chlorure ou bromure.

10. Procédé selon la revendication 3, caractérisé en ce que la catalyseur est ajouté en quantité comprise entre 0,02 et 0,5 équivalent par rapport à chaque groupe carbamate contenu dans le composé de formule II.

11. Procédé selon la revendication 10, caractérisé en ce que le catalyseur est ajouté en quantité comprise entre 0,05 et 0,15 équivalent par rapport audit groupe carbamate.

12. Procédé selon la revendication 1, caractérisé en ce que l'acide halohydrique formé est éliminé du mélange réactionnel par des moyens chimiques ou physiques.

13. Procédé suivant la revendication 12, caractérisé en ce que l'on utilise un accepteur d'acide qui a peu ou pas du tout de pourvoir nucléophile et qui est une base suffisamment forte pour former un complexe avec l'acide halohydrique.

14. Procédé selon la revendication 13, caractérisé en ce que l'accepteur d'acide est choisi dans le groupe constitué par: 1) des 2,4-dialkyl-pyridines et des 2,4,6-trialkyl-pyridines, ledit groupe alkyle contenant entre 1 et n atomes de carbone, n étant un nombre entier suffisamment grand pour que le radical puisse être une chaîne polymère; 2) des N,N-dialkylanilines, non substituées ou substitutées dans le cycle par au moins un groupe électrophile; 3) des alcènes; 4) des diisocyanates qui sont des diisocyanates aliphatiques de formule $O{=}C{=}N{-}(CH_2)_x{-}N{=}C{=}O$, où x est compris entre 6 et 36, ou encore des diisocyanates aromatiques; 5) des carbonates alcalins et alcalino-terreux.

15. Procédé selon la revendication 14, caractérisé en ce que le groupe électrophile dans les dialkylanilines est un atome d'halogène, le diisocyanate est un diisocyanate d'hexaméthylène ou de toluène, l'alcène est un pinène ou un cyclododécatriène.

16. Procédé selon la revendication 12, caractérisé en ce que l'acide halohydrique est éliminé par des moyens physiques, qui sont les suivants:

a) élimination sous vide;

b) élimination sous vide, en utilisant un système à interface importante;

c) passage d'un gaz inerte à travers ou au-dessus du mélange réactionnel;

d) tamis moléculaires.

17. Procédé selon l'une quelconque des revendications précédentes caractérisé en ce que la réaction s'effectue dans un solvant aprotique, qui est non nucléophile ou faiblement nucléophile.

18. Procédé selon la revendication 17, caractérisé en ce que le solvant est choisi dans le groupe constitué par les éthers, les sulfones, les N,N-dialkyl-sulfonamides, les N,N,N',N'-tétraalkylsulfonylurées, les hydrocarbures aromatiques, les hydrocarbures aromatiques ayant un point d'ébullition approprié et au moins un substituant électrophile, les alcanes ou alcènes ayant un point d'ébullition approprié, et les carbamates déshydrohalogénés finaux de formule I.

19. Procédé selon la revendication 18 caractérisé en ce que le solvant est du chlorobenzène, du bromobenzène, du dichlorobenzène, un trichlorobenzène, un tétrachlorobenzène ou le tétrachloroéthylène.

20. Procédé selon la revendication 1, caractérisé en ce que le carbamate alpha-halogéné de formule II réagit en présence d'un catalyseur, qui est un sel facilement ionisable dont l'anion est faiblement nucléophile ou non nucléophile, anisi que d'un accepteur organique de l'acide halohydrique, ledit accepteur ayant peu ou pas de pouvoir nucléophile et étant une base suffisamment forte, capable de complexer l'acide halohydrique formé, et également en présence, d'au moins un solvant, que est un hydrocarbure aromatique substitué par un moins groupe électrophile, un alcane, un alcène, ou les carbamates déshydrohalogénés finaux de formule I.

**Patentansprüche**

1. Verfahren zur Herstellung von Vinylcarbamaten der Formel I:

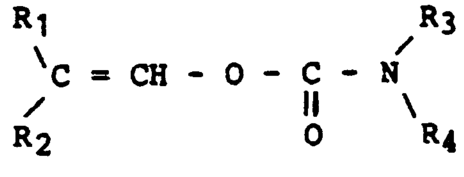

(I)

in der $R_1$ und $R_2$, gleich oder verschieden

ein Wasserstoffatom,

ein aliphatischer, gesättigter oder ungesättigter, substituierter oder nicht substituierter aliphatischer Rest sind oder

zusammen mit dem Kohlenstoffatom, an das sie gebunden sind, einen gesättigten oder ungesättigten, substituierten oder nicht substituierten Ring bilden,

$R^3$ und $R^4$, gleich oder verschieden,

ein Wasserstoffatom,

einen gesättigten oder ungesättigten, substituierten oder nicht substituierten, aliphatischen oder cycloaliphatischen Rest, dessen ungesättigte Bingung sich an einem Kohlenstoffatom befindet, das nicht

neben dem Stickstoffatom ist,

einen Rest der Formel

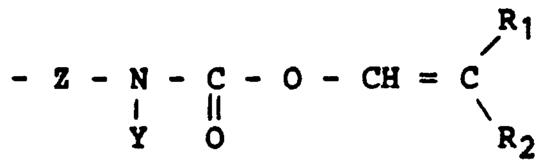

in der Y ein Alkylrest ist,

$R_1$ und $R_2$ wie oben definiert sind und

Z eine Kohlenwasserstoffkette mit 2 bis 20 Kohlenstoffatomen, eine Kohenwasserstoffkette mit 2 bis 20 Kohlenstoffatomen und außerdem mit Heteroatomen W, wobei W O, S, NR'' ist und jedes Heteroatom von einem anderen Heteroatom durch mindestens 2 Kohlenstoffatome getrennt ist und R'' ein Kohlenwasserstoffrest ist, oder eine Gruppe der Formel

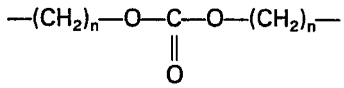

wobei n = eine ganze Zahl von 2 bis 10 ist,

einen substituierten oder nicht substituierten aromatischen Rest bedeuten oder

zusammen mit dem Stickstoffatom, an das sie gebunden sind, einen gesättigten oder ungesättigten, substituierten oder nicht substituierten heterozyklischen Ring bilden,

zusammen mit dem Stickstoffatom, an das sie gebunden sind, einen heterozyklischen Ring bilden, der Teil eines kondensierten, gesättigten oder ungesättigten, substituierten oder nicht substituierten Systems ist.

zusammen mit dem Stickstoffatom, an das sie gebunden sind, einen heterozyklischen Ring mit mindestens einem anderen Heteroatom, wie O, S, N oder $NR_x$, bilden, wobei $R_x$ ein eine Vinylcarbamatgruppe bildender Kohlenwasserstoffrest und/oder Stickstoffatom ist,

zusammen mit dem Stickstoffatom, an das sie gebunden sind, einen Ring der Formel

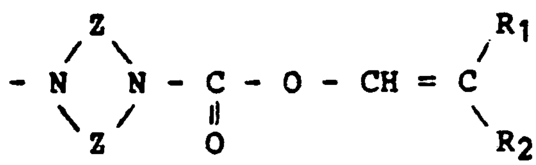

bilden, in der Z, $R_1$ und $R_2$ die oben angegebene Bedeutung haben,

zusammen mit dem Stickstoffatom, an das sie gebunden sind, einen Piperazinring bilden, wobie die beiden Stickstoffatome des Piperazinrings eine Gruppe der Formel

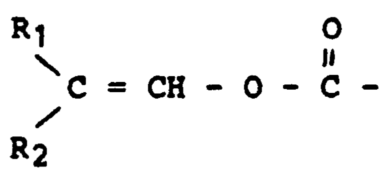

aufweisen, in der $R_1$ und $R_2$ die oben angegebene Bedeutung haben, gekennzeichnet durch Erhitzen auf eine Temperatur von 80 bis 200°C während einigen Minuten bis mehreren Stunden eines α-halogenierten Carbamats der Formel II,

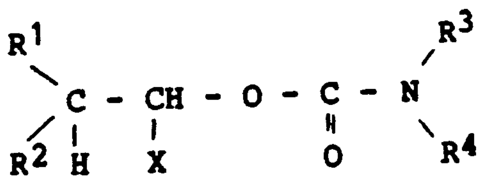

(II)

in der $R_1$, $R_2$, $R_3$ und $R_4$ wie oben definiert sind und, wenn $R_3$ und $R_4$ in der Formel (I), je ein Rest mit einer Vinylcarbamatgruppe ist, dieser in der Verbindung der Formel (II) in Form eines gestättigten Carbamats mit einem Wasserstoffatom in β-Stellung und X in α-Stellung vorliegt, wobei X ein Halogenatom ist.

2. Verfahren nach Anspruch 1, gekennzeichnet durch Verwendung einer Verbindung der Formel II, in der X Chlor oder Brom ist.

3. Verfahren nach Anspruch 1, dadurch gekennzeichnet, daß die Reaktion in Gegenwart eines

Katalysators durchgeführt wird, der ein leicht ionisierbares Salz ist, dessen Anion nicht oder nur wenig nukleophil ist.

4. Verfahren nach Anspruch 3, gekennzeichnet durch Verwendung eines Salzes, dessen Kation unter folgenden Gruppen gewählt wird:

a) Metallkationen,

b) Metallkationen, die mit einem Kronenether verbunden sind,

c) Metallkationen, die mit einem Kryptanden verbunden sind,

d) nicht substituierten Oniumkationen,

e) mit mindestens einem organischen Rest mit 1 bis 7 Kohlenstoffatomen substituierten Oniumkationen.

5. Verfahren nach Anspruch 4, gekennzeichnet durch Verwendung einen Salzes, dessen Kation ein Alkalimetall- oder Erdalkalimetallkation ist.

6. Verfahren nach Anspruch 4, gekennzeichnet durch Verwendung eines Salzes, dessen Oniumkation ein Ammonium-, Phosphonium-, Arsonium- oder Sulfoniumkation ist.

7. Verfahren nach Anspruch 4, gekennzeichnet durch Verwendung eines Salzes, dessen Oniumkation ein Tetra-n-Butylammoniumkation ist.

8. Verfahren nach Anspruch 3, gekennzeichnet durch Verwendung eines Salzes, dessen Anion unter Halogeniden, $ClO_4^-$ und $NO_3^-$ ausgewählt wird.

9. Verfahren nach Anspruch 8, gekennzeichnet durch Verwendung eines Salzes, in dem das Halogenid ein Chlorid oder Bromid ist.

10. Verfahren nach Anspruch 3, durch gekennzeichnet daß der Katalysator in einer Menge von 0,02 bis 0,5 Äquivalenten, bezogen auf jede in der Verbindung der Formel II enthaltenen Carbamatgruppe, zugegeben wird.

11. Verfahren nach Anspruch 10, durch gekennzeichnet, daß der Katalysator in einer Menge von 0,05 bis 0,15 Äquavalenten je Carbamatgruppe zugegeben wird.

12. Verfahren nach Anspruch 1, durch gekennzeichnet, daß die gebildete halogenwasserstoffsäure aus dem Reaktionsgemisch mit chemischen oder physikalischen Mitteln entfernt wird.

13. Verfahren nach Anspruch 12, durch gekennzeichnet, daß ein Säureakzeptor verwendet wird, der wenig oder nicht nukleophil ist und eine ausreichend starke Base zur Bildung eines Komplexes mit der Halogenwasserstoffsäure ist.

14. Verfahren nach Anspruch 13, durch gekennzeichnet, daß der Säureakzeptor under den folgenden Gruppen gewählt wird:

1) 2,4-Dialkyl-pyridinen und 2,4,6-Trialkyl-pyridinen, wobei der Alkylrest von 1 bis n Kohlenstoffatomen aufweist, n eine ausreichend große ganze Zahl ist, damit der Rest eine Polymerkette sein kann,

2) N,N-dialkylanilinen, die gegebenenfalls am Ring mit mindestens einem elektrophilen Rest substituiert sind,

3) Alkenen,

4) Diisocyanaten, wie aliphatischen Diisocyanaten der Formel $O{=}C{=}N{-}(CH_2)_x{-}N{=}C{=}O$ mit X = von 6 bis 36 oder aromatischen Diisocyanaten, und

5) Alkali- oder Erdalkalimetallcarbonaten.

15. Verfahren nach Anspruch 14, gekennzeichnet durch Verwendung von Dialkylanilinen, in denen die elektrophile Gruppe ein Halogenatom ist, von Hexemethylen- oder Toluoldiisocyanat als Diisocyanat und Pinen oder Cyclododecatrien als Alken.

16. Verfahren nach Anspruch 12, dadurch gekennzeichnet, daß die Halogenwasserstoffsäure durch folgende physikalische Mittel entfernt wird:

a) Entfernung unter vermindertem Druck,

b) Entfernung unter vermindertem Druck unter Verwendung eines Systems mit einer bedeutenden Grenzfläche,

c) Leiten eines inerten Gases durch oder über das Reaktionsgemisch und

d) Verwendung von Molekularsieben.

17. Verfahren nach einem der vorstehenden Ansprüche, dadurch gekennzeichnet, daß die Reaktion in einem aprotischen Lösungsmittel, das nicht oder nur gering nukleophil ist, durchgeführt wird.

18. Verfahren nach Anspruch 17, dadurch gekennzeichnet, daß das Lösungsmittel unter Ethern, Sulfonen, N,N-dialkyl-sulfonamiden, N,N,N',N-tetraalkylsulfonylharnstoffen, aromatischen Kohlenwasserstoffen, aromatischen Kohlenwasserstoffen mit einem geeigneten Siedepunkt und mindestens einem elektrophilen Substituenten, Alkanen oder Alkenen mit einem geeigneten Siedepunkt und den dehydrohalogenierten Endcarbamaten der Formel I gewählt wird.

19. Verfahren nach Anspruch 18, dadurch gekennzeichnet, daß das Lösungsmittel unter Chlorbenzol, Brombenzol, Dichlorbenzol, Trichlorbenzol, Tetrachlorbenzol oder Tetrachlorethylen gewählt wird.

20. Verfahren nach Anspruch 1, dadurch gekennzeichnet, daß das α-halogenierte Carbamat der Formel II in Gegenwart eines Katalysators, der ein leicht ionisierbarers Salz ist, dessen Anion gering oder nicht nukleophil ist, eines organischen Akzeptors für die Halogenwasserstoffsäure, wobei der Akzeptor gering oder nicht nukleophil und eine ausreichend starke Base ist, um die gebildete Halogenwasserstoffsäure zu binden, sowie mindestens eines Lösungsmittels umgesetzt wird, das ein mit mindestens einer elektrophilen Gruppe substituierter aromatischer Kohlenwasserstoff, ein Alkan, ein Alken oder ein

dehydrohalogeniertes Endcarbamat der Formel I ist.

**Claims**

1. Process for preparing vinyl carbamates of the formula I:

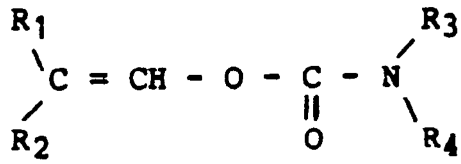 (I)

in which $R_1$ and $R_2$, which may be identical or different, denote

a hydrogen atom, or

a substituted or unsubstituted, saturated or unsaturated aliphatic radical, or alternatively

$R_1$ and $R_2$, together with the carbon atom to which they are attached, form a ring which is saturated or unsaturated and substituted or unsubstituted,

$R_3$ and $R_4$ are identical or different and denote

a hydrogen atom,

an aliphatic or cycloaliphatic radical, substituted or unsubstituted and saturated or unsaturated, the unsaturation occurring on a carbon atom which is not adjacent to the nitrogen atom,

a radical or formula

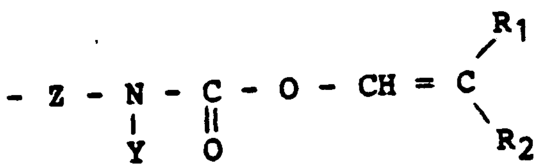

in which Y is an alkyl radical, $R_1$ and $R_2$ are defined as above and Z is a hydrocarbon chain containing 2 to 20 carbon atoms, or Z is a hydrocarbon chain consisting of 2 to 20 carbon atoms and containing, in addition, hetero atoms W, W being O, S, NR'', each hetero atom being separated from another hetero atom by at least two carbon atoms and R'' being a hydrocarbon radical, or Z is a group

$$-(CH_2)_n-O-\underset{\underset{O}{\|}}{C}-O-(CH_2)_n-$$

10, or

a substituted or unsubstituted aromatic radical, or alternatively

$R_3$ and $R_4$ form, together with the nitrogen atom to which they are attached, a substituted or unsubstituted, saturated or unsaturated heterocycle,

$R_3$ and $R_4$ form, together with the nitrogen atom to which they are attached, a heterocycle which is part of a substituted or unsubstituted, saturated or unsaturated fused ring system,

$R_3$ and $R_4$ form, together with the nitrogen atom to which they are attached, a heterocycle which contains at least one other hetero atom which is O, S, N or $NR_x$, $R_x$ being a hydrocarbon radical, and/or a nitrogen atom which forms a vinyl carbamate group,

$R_3$ and $R_4$ form, together with the nitrogen atom to which they are attached, a ring of formula

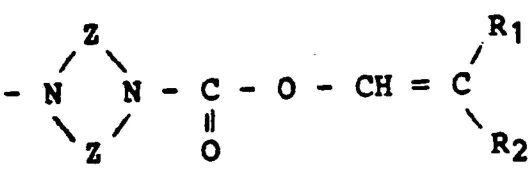

in which Z, $R_1$ and $R_2$ have the above meaning,

$R_3$ and $R_4$ form a piperazine ring together with the nitrogen atom to which they are attached, the two nitrogen atoms of the piperazine ring bearing the group of formula:

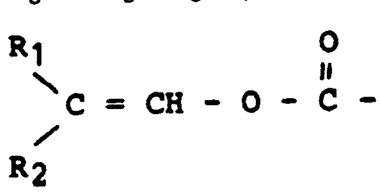

in which $R_1$ and $R_2$ have the above meaning,

which consists in heating an alpha-halogenated carbamate of formula (II):

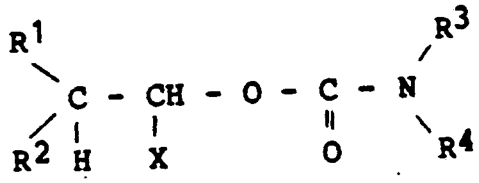

(II)

in which $R_1$, $R_2$, $R_3$ and $R_4$ are defined as above and when, in the compound of formula (I), $R_3$ and $R_4$ denote a radical which contains a vinyl carbamate group, the latter is present in the compound of the formula (II) in the form of a saturated carbamate containing a hydrogen atom at the beta-position and X at the alpha-position, X is a halogen atom, at a temperature of between 80°C and 200°C for a period of between a few minutes and several hours.

2. Process according to Claim 1, in which X is chlorine or bromine.

3. Process according to Claim 1, characterized in that the reaction is performed in the presence of a catalyst which is readily ionizable salt, the anion of which is non-nucleophilic or weakly nucleophilic.

4. Process according to Claim 3, characterized in that the cation of the said salt is chosen from the group consisting of: a) a metal cation; b) a metal cation complexed with a crown ether; c) a metal cation complexed with a cryptand; d) an unsubstituted onium cation; e) an onium cation substituted with at least one organic radical having from 1 to 7 carbon atoms.

5. Process according to Claim 4, characterized in that the cation is an alkali metal or alkaline earth metal cation.

6. Process according to Claim 4, characterized in that the onium cation is an ammomium, phosphonium, arsonium or sulphonium cation.

7. Process according to Claim 4, characterized in that the onium cation is a tetra-n-butylammonium cation.

8. Process according to Claim 3, characterized in that the anion is selected from the group consisting of halides, $ClO_4^-$ and $NO_3^-$.

9. Process according to Claim 8, characterized in that the halide is a chloride or bromide.

10. Process according to Claim 3, characterized in that the catalyst is added in an amount between 0.02 and 0.5 equivalent relative to each carbamate group present in the compound of formula II.

11. Process according to Claim 10, characterized in that the catalyst is added in an amount between 0.05 and 0.15 equivalent relative to the said carbamate group.

12. Process according to Claim 1, characterized in that the hydrohalic acid formed is removed from the reaction mixture by chemical or physical means.

13. Process according to Claim 12, characterized in that an acceptor for acid which has little or no nucleophilic power, and which is a sufficiently strong base to form a complex which the hydrohalic acid, is used.

14. Process according to Claim 13, characterized in that the acceptor for acid is selected from the group consisting of: 1) 2,4-dialkylpyridines and 2,4,6-trialkylpyridines, the said alkyl group containing between 1 and n carbon atoms, n being a sufficiently large integer for the radical to be able to be a polymer chain; 2) N,N-dialkylanilines, unsubstituted or substituted in the ring with at least one electrophilic group; 3) alkenes; 4) diisocyanates which are aliphatic diisocyanates of formula $O{=}C{=}N{-}(CH_2)_x{-}N{=}C{=}O$, where x is between 6 and 36, or alternatively aromatic diisocyanates; 5) alkali metal and alkaline earth metal carbonates.

15. Process according to Claim 14, characterized in that the electrophilic group in the dialkylanilines is a halogen atom, the diisocyanate is a hexamethylene or toluene diisocyanate and the alkene is a pinene or a cyclododecatriene.

16. Process according to Claim 12, characterized in that the hydrohalic acid is removed by physical means which are as follows:

a) removal under vacuum;

b) removal under vacuum using a large-interface system;

c) passage of an inert gas through or above the reaction mixture;

d) molecular sieves.

17. Process according to any one of the preceding claims, characterized in that the reaction is performed in an aprotic solvent which is non-nucleophilic or weakly nucleophilic.

18. Process according to Claim 17, characterized in that the solvent is selected from the group consisting of ethers, sulphones, N,N-dialkylsulphonamides, N,N,N',N'-tetraalkylsulphonylureas, aromatic hydrocarbons, aromatic hydrocarbons having a suitable boiling point and at least one electrophilic substituent, alkanes or alkenes having a suitable boiling point and the final dehydrohalogenated carbamates of formula I.

19. Process according to Claim 18, characterized in that the solvent is chlorobenzene, bromobenzene, dichlorobenzene, a trichlorobenzene, a tetrachlorobenzene or tetrachloroethylene.

20. Process according to Claim 1, characterized in that the alpha-halogenated carbamate of formula II reacts in the presence of a catalyst which is a readily ionizable salt, the anion of which is weakly nucleophilic or non-nucleophilic, as well as of an organic acceptor for the hydrohalic acid, the said acceptor having little or no nucleophilic power and being a sufficiently strong base, capable of complexing the hydrohalic acid formed, and also in the presence of at least one solvent which is an aromatic hydrocarbon substituted with at least one electrophilic group, an alkane, an alkene or the final dehydrohalogenated carbamates of formula I.